# EUROPEAN PATENT SPECIFICATION

(11) **EP 0 738 499 B1**
(45) Date of publication and mention of the grant of the patent: **12.04.2000**
(21) Application number: 96111375.0
(22) Date of filing: 17.07.1990
(51) Int. Cl.: A61B 17/128

(54) **Apparatus for applying surgical clips in laparoscopic or endoscopic procedures**
Vorrichtung zum Anbringen von chirurgischen Klammern bei laparoskopischen oder endoskopischen Eingriffen
Dispositif pour l'application des agrafes chirurgicales dans les procédures de laparoscopie ou d'endoscopie

(30) Priority: 18.07.1989 US 381265; 30.05.1990 US 530652
(43) Date of publication of application: 23.10.1996
(62) Divisional of application: 90307811.1
(73) Proprietor: United States Surgical Corporation, Norwalk, Connecticut 06856 (US)
(72) Inventor: Green, David T., Westport, CT 06880 (US); Bolanos, Henry, East Norwalk, CT 06855 (US); Young, Wayne P., Brewster, NY 10509 (US); McGarry, Richard A., Norwalk, CT 06851 (US); Heaton, Lisa M., Norwalk, CT 06850 (US); Ratcliff, Keith, Sandy Hook, CT 06482 (US)
(74) Representative: Marsh, Roy David

(56) References cited:
- EP-A- 0 170 592
- EP-A- 0 207 480
- EP-A- 0 220 643
- US-A- 4 027 510

## Description

### BACKGROUND OF THE INVENTION

### 1. Field of the Invention

This invention relates to an apparatus for applying surgical clips, especially hemostatic clips, to body tissue such as blood vessels. More particularly, this invention relates to a surgical clip applier which can be used in laparoscopic or endoscopic procedures. Such an instrument is disclosed in US-A-4,027,510, which corresponds to the pre-characterising part of claim 1 below.

### 2. Background of the Related Art

In surgical operations it is often necessary to apply hemostatic clips to blood vessels, and apparatus for applying clips are known in the art. See, for example, U.S. Patent No. 4,616,650, and 4,624,254 and EP-A-0 207 480 which disclose a surgical clip applying apparatus having a pair of ring-like handles. The handles are squeezed to force jaws to move distally relative to the apparatus where they are forced together by a pair of inclined surfaces. A surgical clip between the jaws is thereby squeezed closed.

In laparoscopic procedures surgery is performed through a small incision; in endoscopic procedures surgery is performed through narrow endoscopic tubes inserted through small entrance wounds in the skin. Laparoscopic and endoscopic procedures generally require that any instrumentation inserted into the body be sealed, i.e., provisions must be made to ensure that gases do not enter or exit the body through the laparoscopic or endoscopic incision as, for example, in surgical procedures in which the surgical region is insufflated. Moreover, laparoscopic and endoscopic procedures often require the surgeon to act on organs, tissues, and vessels far removed from the incision, thereby requiring that any instruments to be used in such procedures be both long and narrow. Up to now there have been no instruments for placing a plurality of surgical clips in succession in laparoscopic or endoscopic procedures.

Because endoscopic procedures are more common than laparoscopic procedures, the present invention shall be discussed in terms of endoscopic procedures and apparatus. However, use herein of terms such as "endoscopic", "endoscopically" and "endoscopic portion", among others, refer generally to instruments having elongated and relatively narrow operating portions for inserting into a cannula or a small wound in the skin and should not be construed to limit the present invention to an apparatus for applying surgical clips only in conjunction with an endoscopic tube. To the contrary, it is believed that the present invention may find use in any procedure where access is limited to a small incision, including, but not limited to laparoscopic procedures.

### 3. Objects of the Invention

Accordingly, it is one object of the present invention to provides a surgical clip applier, for placing a plurality of clips in succession and which can be used endoscopically, and which is adapted to prevent gases from communicating between the interior and exterior of the body during an endoscopic procedure.

### SUMMARY OF THE INVENTION

In accordance with the present invention, there is provided an apparatus as defined in claim 1 below. The invention provides apparatus for applying surgical clips to body tissue which comprises frame means, endoscopic means connected to the frame means of generally elongated configuration and extending distally from the frame means and including means for storing a plurality of surgical clips, means for selectively advancing the clips to the distal portion of the endoscopic means for positioning adjacent the body tissue to be clipped; and means for at least partially closing each clip at least sufficient to grip the body tissue after the clip has been advanced distally to the predetermined portion of the endoscopic means.

In a preferred embodiment, the apparatus for applying surgical clips to body tissue comprises a frame, and an elongated endoscopic section connected at the proximal end thereof to the frame and extending distally from the frame. The endoscopic section includes means for storing a plurality of surgical clips and a pair of jaws positioned at the distal portion of the endoscopic section and adapted for reception of the clips. Means is provided for sequentially advancing the clips distally for positioning within the pair of jaws to be positioned adjacent the body tissue to be clipped; and means is provided for sequentially at least partially closing the jaws about the clips to close the clips at least partially about the body tissue.

In one embodiment, a completely disposable apparatus is disclosed for applying surgical clips to body tissue which comprises a frame configured and dimensioned for manual gripping, an elongated endoscopic section connected at the proximal end thereof to the frame and extending distally therefrom, the endoscopic section including means for storing a plurality of surgical clips in generally aligned relation facing the distal portion thereof, jaw means positioned at the distal end thereof and adapted for sequential reception of the clips, means for sequentially advancing the clips distally as to be positioned between the jaw means for positioning adjacent the body tissue to be clipped, and means for sequentially at least partially closing the jaw means about each clip after the clip is advanced therebetween while simultaneously repositioning the clip advancing means for distal advancement of the next clip.

In another embodiment of the present invention the endoscopic portion is formed as a disposable unit detachable from a reusable frame and handle portion.

Preferably, an instrument body is provided and an actuating handle mounted to the instrument body, with first transmission means for linearly transferring motion from the actuating handle to the clip advancing means and means to close the jaw means. Second transmission means is provided for linearly transferring motion from the actuating handle to the jaw closing means, and means is provided for locking the handle such that after actuating the handle to close the jaws the handle cannot be actuated unless the locking means is released. The endoscopic section is rotatable independent of the handle, with means being provided to selectively lock the endoscopic section at a predetermined angular orientation relative to the handle. Means is provided to release the lock means of the endoscopic section so as to permit rotation thereof relative to the handle.

Handle locking means comprises a first resilient catch movable in response to actuation of the handle from an unlocked position to a locked position wherein the first transmission means is advanced and locked. Release means is adapted to release the first resilient catch, the first resilient catch being returnable to the unlocked position in response to actuation of the release means. A second resilient catch is movable in response to actuation of the handle from an unlocked position to a locked position wherein it engages and locks the second transmission means. The second resilient catch is resiliently returnable to the unlocked position in response to the release of the resilient catch. The first transmission means comprises means responsive to actuation of the release means to release the second transmission means. The jaw means preferably comprises a pair of jaws positioned in spaced relation and configured and dimensioned for reception of a surgical clip therebetween. The jaws are resiliently movable toward and away from each other in response to distal movement of a camming means from a proximal position to a distal position. The camming means comprises a channel member slidably mounted within the endoscopic section and longitudinally movable in response to actuation of the handle. The channel member having at least two distal camming surfaces for biasing the jaws into the closed position. Means for storing surgical clips comprises a track for holding a longitudinal array of surgical clips, and resilient means located proximal to the array of surgical clips for biasing the surgical clips toward the distal direction. A clip track is positioned between the jaw means and the clip follower. Means for advancing the surgical clips comprises a pusher bar for advancing the distal-most clip in the area of the pair of jaws, the pusher bar being longitudinally slidable in response to actuation of the handle, and escapement means located at the distal end of the array of clips for preventing more than one clip at a time from being advanced into the jaw means. The escapement means comprises a plurality of projections upstanding from the clip track and extending into the clip path.

The first transmission means comprises a pusher bar, and a proximal pusher tube connected to the proximal end of the pusher bar. The pusher bar is movable between a first position wherein the distal end of the pusher bar is located proximally of the surgical clip to be advanced, and a second position wherein the distal end of the pusher bar advances the surgical clip to the jaw means. The first pusher tube includes mounting means for rotatably connecting the pusher bar thereto. The mounting means of the pusher tube comprises a generally circular shaped projection dimensioned for reception and engagement of at least one cooperating projection on the pusher bar.

The second transmission means comprises a channel member positioned within the endoscopic section, a proximal channel tube connected to the proximal end portion of the channel member, and a channel tube adapted for rotatably connecting the channel member thereto. The channel member is connected to the camming means for closing the jaw means.

The jaw means preferably comprises a jaw blade fixed to the endoscopic section and having a pair of distal spaced jaws which are resiliently movable between a closed position for closing a surgical clip and an open position for reception of the surgical clip. The camming means is comprised of a channel member having camming surfaces movable from a first position proximal of the jaws, and a second distal position wherein the camming surfaces of the channel member move the jaws to the closed position. The channel member is connected at its proximal end to the channel tube.

The rotatable mounting means of the channel tube comprises a circumferential projection dimensioned for engaging at least one cooperating notch in the camming means. The endoscopic section is rotatable about a longitudinal axis extending relative to the frame between a plurality of click-stop settings. Further, the endoscopic section is preferably adapted to provide a gaseous seal means, optionally, in the form of silicone grease.

With the apparatus of the present invention the endoscopic portion of the apparatus can be inserted into the body through a small incision or, more likely, through an endoscopic tube. The blood vessel or other tissue to be clipped can be engaged by the jaws of the apparatus. A clip can be positioned between the jaws and the jaws then closed, thereby applying the clip to the blood vessel.

The present invention advantageously permits a surgeon to perform internal application of a plurality of clips in succession, without full access to the operation site, i.e., without providing a large opening in the body to allow access to the operation site. The frame and handle portion of the apparatus are manipulated outside of the patient's body. Additionally the endoscopic portion may be rotated so as to facilitate positioning of the clip.

The ability to apply surgical clips through a small incision or tube dramatically reduces blood loss, tissue trauma, and patient recovery time, thereby contributing to improved health care practices.

### BRIEF DESCRIPTION OF THE DRAWINGS

Preferred embodiments of the invention are described hereinbelow with reference to the drawings wherein:
Fig. 1 is a perspective view of a completely disposable apparatus for placing clips in laparoscopic or endoscopic procedures constructed according to the present invention;
Fig. 2 is a perspective view with parts separated for purposes of illustration of the endoscopic section of the apparatus of Fig. 1;
Fig. 3 is a perspective view with parts separated for purposes of illustration of the handle section of the apparatus of Fig. 1 utilized for activating the endoscopic section;
Fig. 4 is a plan view from above, of the distal portion of the endoscopic section of the apparatus of Fig. 1;
Fig. 5 is a cross-sectional view taken along lines 5-5 of Fig. 4 illustrating the clip pusher in position to push the clip next in line to a position between the jaws of the clamp of the apparatus;
Fig. 5a is a cross-sectional view of the distal portion of the endoscopic section of the apparatus of Fig. 1, illustrating the position of the clip pusher after the last clip has been advanced into the jaws of the endoscopic section;
Fig. 5b is a cross-sectional view taken along lines 5b-5b of Fig. 5a;
Fig. 6 is a plan view from above similar to Fig. 4 of the distal portion of the endoscopic section of the apparatus with the clip pusher in the distal position with the clip shown in Fig. 5 now positioned between the jaws of the clamp;
Fig. 7 is a cross-sectional view taken along lines 7-7 of Fig. 6;
Fig. 8 is a schematic view of the jaws positioned around the cystic duct;
Fig. 9 is a cross-sectional view taken along lines 9-9 of Fig. 6;
Fig. 10 is a cross-sectional view taken along lines 10-10 of Fig. 6;
Fig. 11 is a cross-sectional view taken along lines 11-11 of Fig. 6;
Fig. 12 is a plan view from above of the distal portion of the endoscopic section illustrating a clip positioned within the clamp jaws after clamping is completed about an artery;
Fig. 13 is a side view thereof;
Fig. 14 is a cross-sectional view taken along lines 14-14 of Fig. 12;
Fig. 15 is a cross-sectional view taken along lines 15-15 of Fig. 12;
Fig. 16 is an elevational cross-sectional view of the handle of the apparatus of Fig. 1 illustrating the channel tube in the normal "at rest" position and the pusher tube in the loaded position corresponding to the position of the clip pusher in the proximal position about to push the next clip distally into the jaws;
Fig. 17 is an elevational cross-sectional view of the handle of the apparatus of Fig. 1 with the pusher tube extended to its distal-most position corresponding to the position of the clip pusher after positioning a clip between the jaws, and the channel tube in its proximal position corresponding to open jaws at the distal-most position of the endoscopic section;
Fig. 18 is an elevational cross-sectional view of the handle section of the apparatus of Fig. 1 illustrating the pusher tube in its proximal position corresponding to the clip pusher in position behind the next clip to be advanced and the channel tube in its distal-most position after having closed the jaws of the clamp;
Fig. 19 is an elevational cross-sectional view of the handle of Fig. 1 illustrating the channel tube in locked position after the last clip has been pushed into the jaws of the endoscopic section and the pusher bar has engaged a distal slot in the clip follower, locking the pusher tube in the position shown;

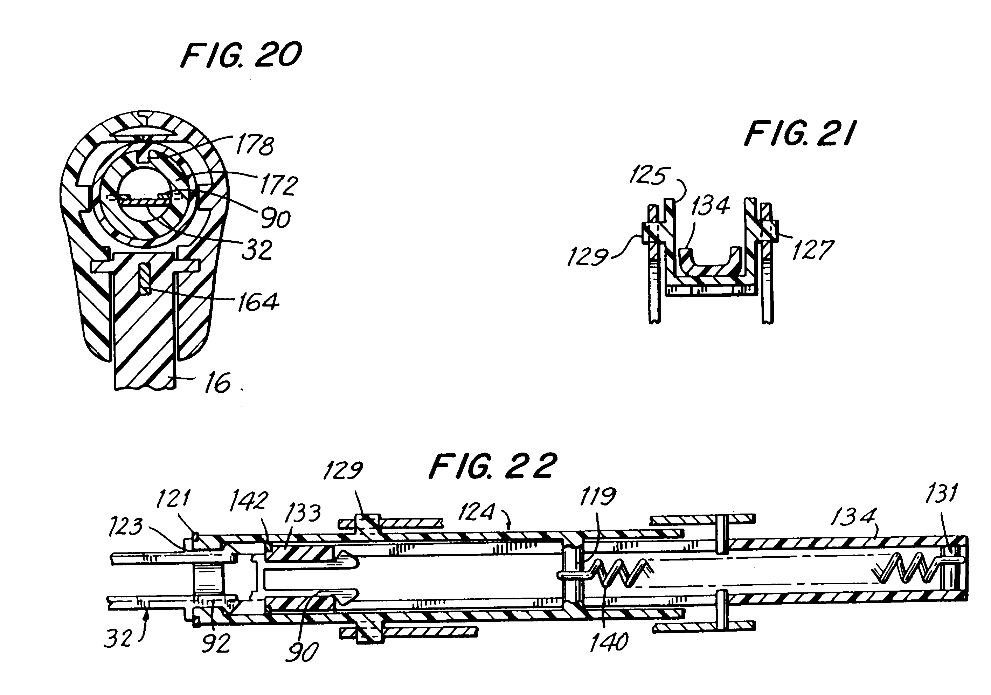

Fig. 20 is a cross-sectional view taken along lines 20-20 of Fig. 17 and illustrating the channel tube and pusher bar;
Fig. 21 is a view taken along lines 21-21 of Fig. 17 illustrating the pusher tube and the channel tube in cross-section;
Fig. 22 is a cross-sectional view taken along lines 22-22 of Fig. 17 illustrating the pusher tube mainspring and the connection between the pusher tube and the pusher bar;

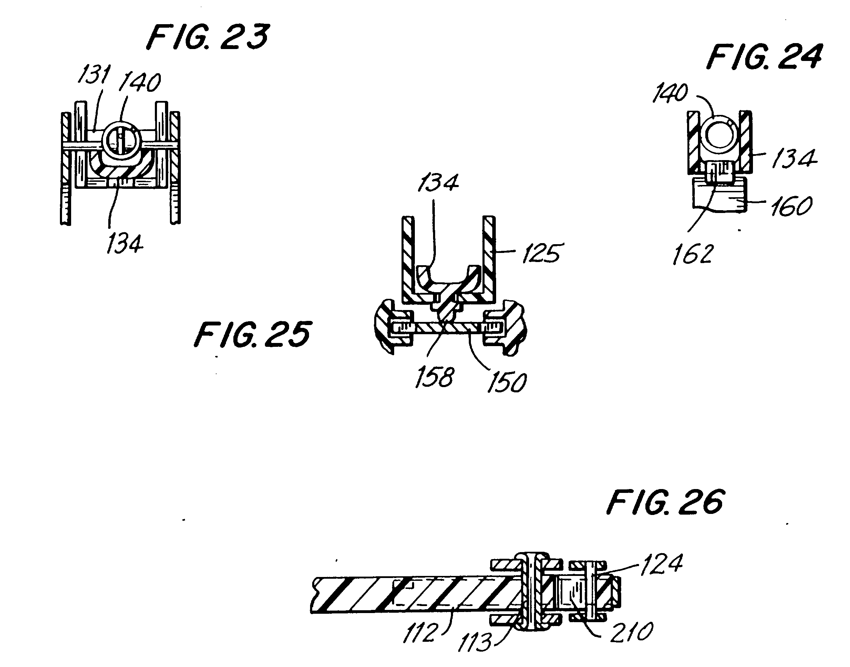

Fig. 23 is a cross-sectional view taken along lines 23-23 of Fig. 17 illustrating the pusher tube and the pusher tube link pin;
Fig. 24 is a cross-sectional view taken along lines 24-24 of Fig. 17, illustrating the pusher tube and the mainspring;
Fig. 25 is a cross-sectional view taken along lines 25-25 of Fig. 17 illustrating release of the channel tube latch plate by the pusher tube; and
Fig. 26 is a cross-sectional view taken along lines 26-26 of Fig. 17 illustrating the lost motion spring which permits partial clamping of the surgical clips.
Fig. 27 illustrates a cutaway perspective view of a second embodiment of the present invention;
Fig. 28 illustrates a perspective view of the frame portions of the second embodiment;
Fig. 29 illustrates an exploded perspective view of the actuating mechanism of the second embodiment;
Fig. 30 illustrates a perspective view of the handle;
Fig. 31 illustrates a side view of the right toggle lever;
Fig. 32 illustrates a perspective view of the left toggle lever;
Fig. 33 illustrates a side view of the pusher bar front link;
Fig. 34 illustrates a side view of the pusher bar rear link;
Fig. 35 illustrates a perspective view of the rear channel;
Fig. 36 illustrates a perspective view of the rear pusher bar;
Fig. 37 illustrates a perspective view of the pusher bar stop spring;
Fig. 38 illustrates a front view of the pusher slide;
Figs. 39a and 39b illustrate a front view of the channel lock pin in the unlocked and locked positions, respectively;
Fig. 40 illustrates the channel lock pin in perspective view;
Fig. 41 illustrates an exploded perspective view of the transmission mechanism of the second embodiment;
Fig. 42 illustrates a sectional top view of the transmission coupling;
Fig. 43 illustrates a sectional top view of the collar fitting;
Fig. 44 illustrates an exploded perspective view of the endoscopic portion of the second embodiment;
Figs. 45a and 45b illustrate in perspective view the closing of the jaw blade to apply a surgical clip;
Fig. 46 illustrates a cutaway perspective view of an alternative embodiment;
Fig. 47 illustrates the frame of an alternative embodiment;
Fig. 48 illustrates an exploded perspective view of the actuating and transmission system of an alternative embodiment;
Fig. 49 illustrates the handle of an alternative embodiment;
Figs. 50a and 50b illustrate in sectional side view and bottom view respectively, the pusher tube of an alternative embodiment;
Figs. 51 and 51a illustrate the channel tube of an alternative embodiment in perspective and side sectional views, respectively.
Fig. 52 illustrates the leaf spring of an alternative embodiment;
Fig. 53 illustrates the release button of an alternative embodiment;
Fig. 54 illustrates a sectional side view of the outer tube of an alternative embodiment;
Fig. 55 illustrates in exploded view the endoscopic portion of an alternative embodiment;
Fig. 56 illustrates a sectional top view of the collet and sleeve of an alternative embodiment;
Fig. 57 illustrates a sectional side view of the upper portion of the cartridge of an alternative embodiment;
Fig. 58 illustrates a sectional side view of the lower portion of the cartridge of an alternative embodiment;
Figs. 59a and 59b illustrate the proximal section of the endoscopic portion of an alternative embodiment;
Figs. 60a and 60b illustrate the endoscopic portion of the apparatus used in conjunction with the cannula of a trocar in top and side views, respectively;

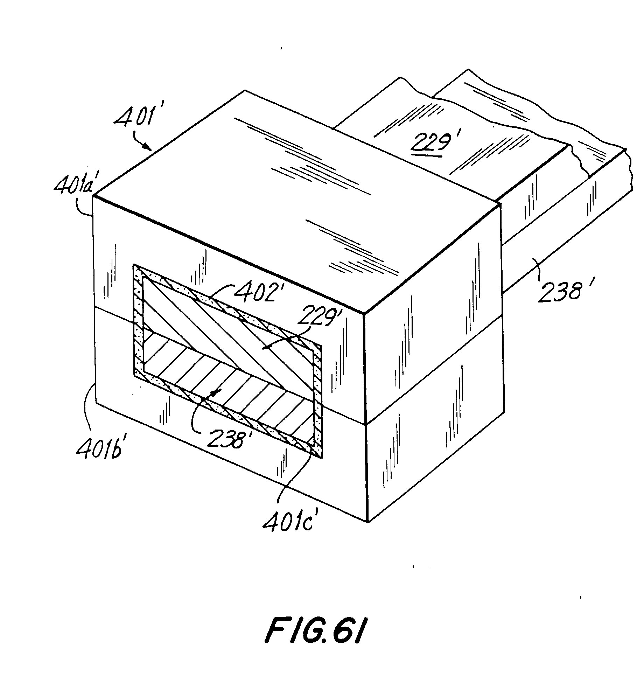

Fig. 61 illustrates the sealing block in perspective view.

### DETAILED DESCRIPTION OF THE INVENTION

The surgical apparatus described herein is adapted to apply surgical clips to blood vessels and the like, in endoscopic or laparoscopic procedures.

The apparatus or instrument generally comprises a frame which is of a size convenient for being held in the hand and which houses the non-endoscopic body of the apparatus. An endoscopic portion defining a longitudinal axis extends distally from the frame and is rotatable around the longitudinal axis relative to the frame. The endoscopic portion is a long tube-like portion having a relatively narrow outer diameter (e.g., about 10 millimeters) for insertion into an endoscopic tube such as a trocar cannula, or a small incision.

The endoscopic portion comprises means for holding a longitudinal array of surgical clips, such as a track, with a spring means to bias the clips forward in the distal direction. The clips are generally U-shaped pieces of integral construction and comprise two spaced apart legs connected by a bridge portion. The endoscopic portion has a clip closing means comprising a pair of flexible opposing jaws which are cammed together into closure by a distally-moving channel, and means such as a spring mounted pusher bar for advancing the surgical clips one at a time to the jaws.

The apparatus further has actuating means such as a pivoting handle and connecting links and levers, and transmission means for transferring the pivotal movement of the actuating means linearly along the instrument axis to both the clip closing and clip advancing means. The apparatus also includes tubular members with circumferential coupling means such as circumferential notches or projections, which allow a connection of the endoscopic portion to the frame such that linear actuation movement may be transmitted thereto while allowing the endoscopic portion to rotate around the instrument axis.

The apparatus further comprises a locking means such that once the jaw means have been actuated and opened, the apparatus cannot be reactuated until the locking means is released, usually by a release button. The locking means comprises a resilient catch (such as a catch in conjunction with a spring) which is movable in response to actuation of the apparatus, i.e., application of a clip, from an unlocked position to a locked position. In the locked position, the locking means is engaged, thereby preventing linear transfer of movement to the clip closing means by the transmission means. Preferably two spring catches are used, one for locking the transmission means for actuating the clip advancing mechanism (e.g., pusher bar), and a second catch for locking the transmission means for actuating the jaw closing means (e.g., the channel). Pressing the release button releases both the first and second catches. The first catch is released directly thereby allowing the first transmission means to slide forward, and the second catch is released in response to the forward movement of the first transmission means.

As mentioned above, endoscopic instrumentation is usually required to have a gaseous seal to prevent communication of gases through the endoscopic incision. This gaseous seal may be accomplished in the apparatus of the present invention by providing close tolerances between the outer diameter of the endoscopic portion and the inner diameter of the trocar cannula through which it is inserted, and by providing close tolerances for the internal moving parts of the endoscopic portion. Thus adapted, the instrument of the present invention will provide a suitable gaseous seal.

The instrument of the present invention has four basic actions or functions. First, the endoscopic portion is introduced into the body and positioned with the jaws engaging the blood vessel to be clipped. This may involve rotation of the endoscopic portion relative to the body, either by rotating the apparatus as a whole, or by rotating the endoscopic portion relative to the frame, or by a combination of both actions.

The second action is unlocking the instrument and positioning a clip between the jaws.

Third, the instrument has a means for applying a surgical clip to a blood vessel or other tissue. This may be accomplished by a camming and clamping action. With a surgical clip in position between the jaws of the instrument and the jaws and clip surrounding a blood vessel, a channel member is moved distally which cams the jaws closed and thereby clamps the surgical clip onto the blood vessel.

The fourth action is that of locking the instrument after a clip has been applied and the jaws opened so that the jaws cannot inadvertently be closed again without further action, e.g., pressing a button to feed a new clip.

After the clipping operation has been completed the instrument may be removed from the body. In one embodiment of the present invention the entire instrument may be discarded. In another embodiment the endoscopic portion may be detached and discarded, and the frame and handle portion may be retained for a subsequent reuse with a replacement of the endoscopic portion.

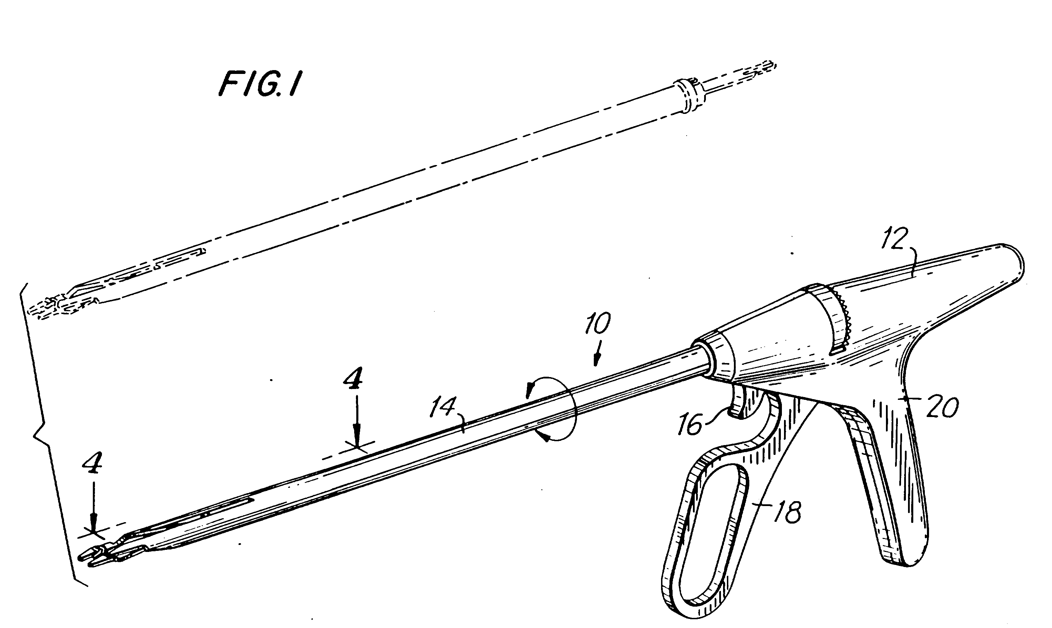

Referring initially to Fig. 1, the apparatus for applying clips in endoscopic and laparoscopic procedures is disclosed in perspective view. The apparatus is preferably constructed as a disposable item of several materials as will be described. Essentially, however, two basic materials are used, i.e., a polycarbonate material such as LEXAN brand plastic material by General Electric Company and stainless steel. Other suitable materials are contemplated.

Briefly, the apparatus 10 includes two main sections. A handle section 12 and an endoscopic section 14 which is distal of the handle section. A clip pushing system which will be described hereinbelow is operative by single finger operative trigger 16 and a clip clamping mechanism is operative by squeezing handle 18 toward hand grip 20 using multiple fingers of the operator.

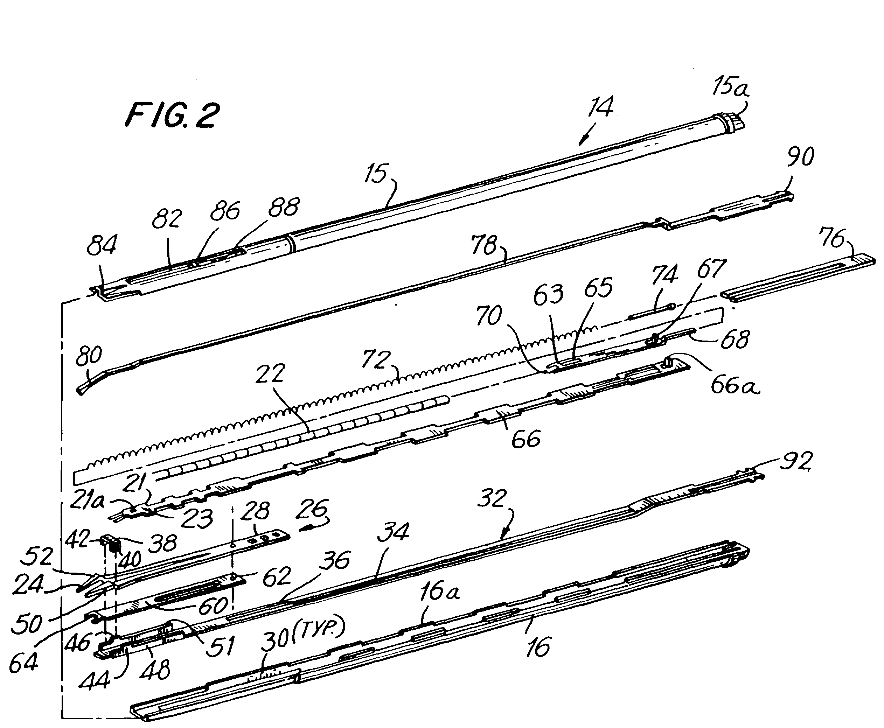

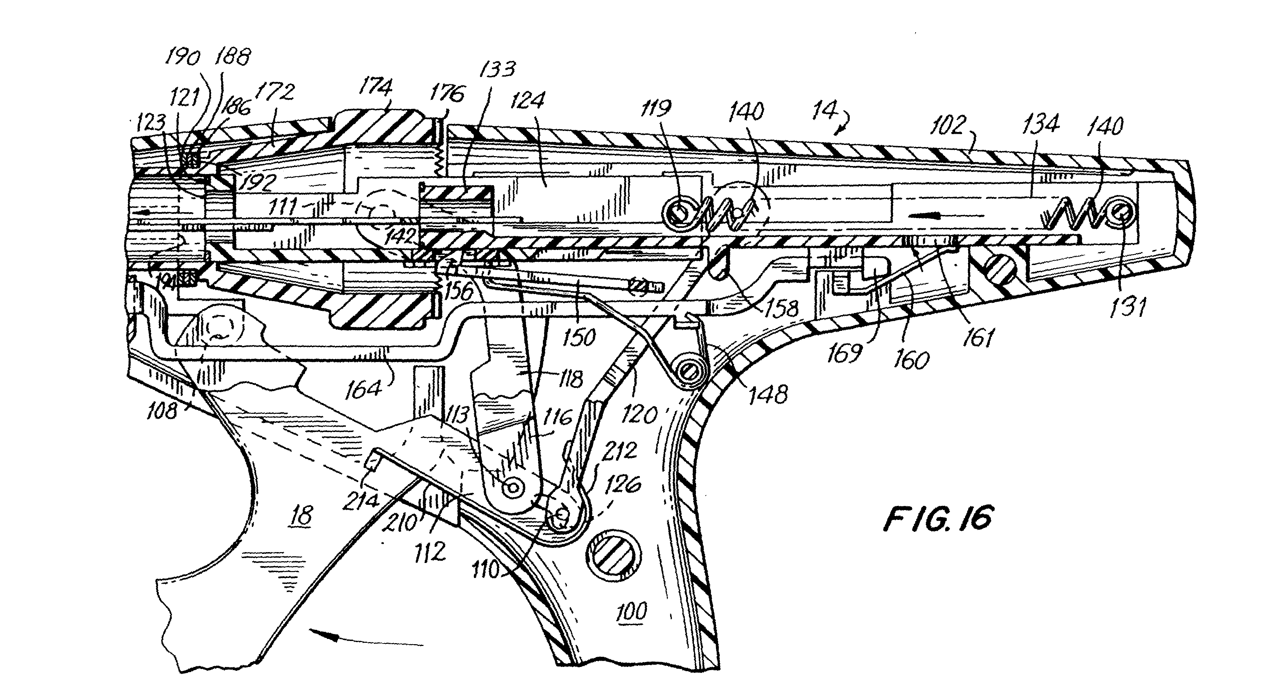

Referring now to Figs. 2 and 4-14 in conjunction with Fig. 1, the endoscopic section 14 of the apparatus 10 will now be described. The endoscopic section 14 is preferably housed in a non-removable cartridge formed of upper half section 15 and lower half section 17. Each half section is formed of a material capable of withstanding the stresses applied by the inner working compartments without deformation or compromise of precision. A polycarbonate material such as LEXAN brand material marketed by General Electric Corporation has been found to satisfy the requisite strength and deformation requirements. Other suitable materials may be used. If desired, the cartridge can be constructed to be removable from the handle.

The lower housing half section 17 includes upstanding tabs 17a and the upper housing half section 15 includes correspondingly positioned slots (not shown) which are dimensioned to receive tabs such that the two half sections may be attached by ultrasonic welding techniques. Preferably, the slots are dimensioned to receive the tabs in interference relation to assist securement of the half portions together. Alternatively, the half sections may be adhesively attached. Further, upper half section 15 includes longitudinally extending slots which receive correspondingly dimensioned ribs in the collar of the handle section (to be described) to facilitate rotation of the endoscopic section with the collar.

Referring once again to Fig. 2, a plurality of U-shaped clips 22 are positioned within the housing for movement in the distal direction in preparation for the clamping procedures. The clips are preferably of titanium for use in clipping blood vessels such as arteries. This material avoids the "starburst" effect and facilitates enhanced CT imaging. The clips 22 are aligned in a row as shown, with the leg portions facing distally. A jaw blade 26 is positioned at the distal end and includes a pair of jaws 24 for reception of each clip whereby the jaws are brought together to close the clip about the artery.

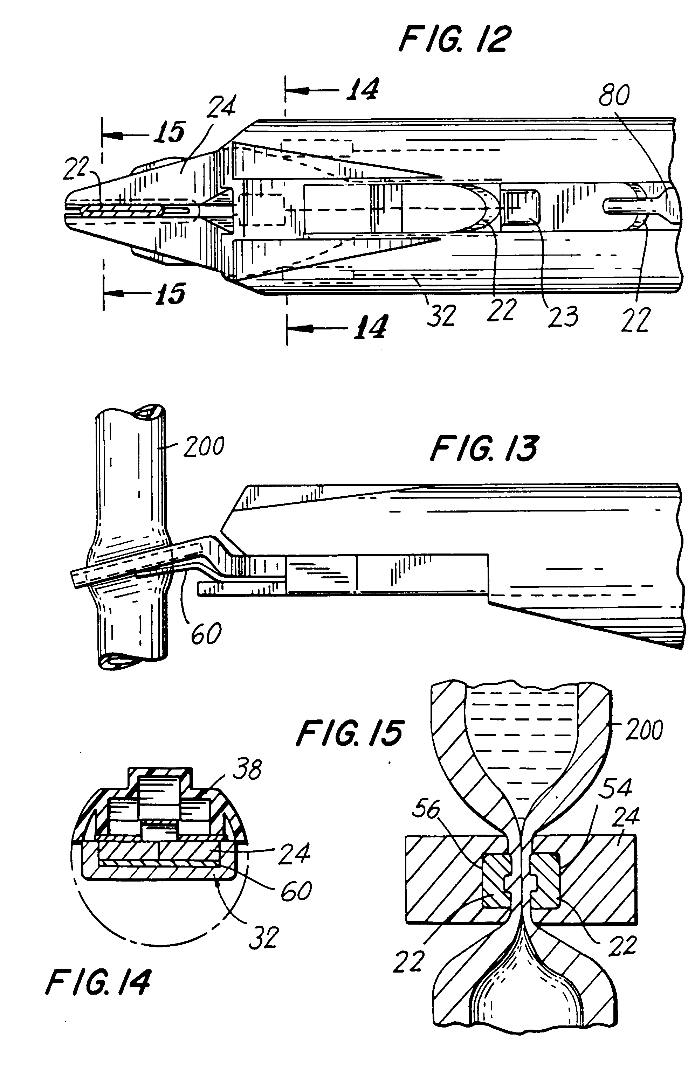

The basic objective is to bias the clips toward the distal direction and to sequentially advance each clip into the jaws after the jaws have been positioned about an artery. Thereafter, the jaws are closed and both legs of the "U" shaped clip are brought together to just sufficiently close the artery as shown in Fig. 12.

The jaw blade 26 is fabricated of a material having sufficient resilience such that clamping of the distal pair of jaws 24 toward each other to close a clip therebetween will be followed by return of the jaws to their original position upon release of the clamping forces. Stainless steel has been found to be a preferred material capable not only of withstanding the requisite number of clamping cycles without adverse affect, but also of being suitably sterilized. Furthermore, jaw blade 26 includes three square shaped apertures 28 dimensioned to receive three correspondingly shaped pins 30 molded into the lower body half section 16 of the housing to position the jaw blade 26 with respect to the body.

Referring further to Fig. 2, crimping channel 32 is dimensioned and positioned for slidable movement within the body of the housing and defines elongated slot 34 having a wider portion 36 at the distal end for reception of square pins 30. The width of the slot 34 in distal portion 36 of crimping channel 32 is just sufficient to receive the pins 30 to maintain relative alignment between the jaw blade 26 and the pins 30. A channel bracket 38, also preferably of stainless steel, is positioned atop the jaw blade and defines two downwardly extending side walls 40, 42 positioned to be welded to the distal portions of correspondingly positioned and dimensioned upwardly extending side walls 48, 50 of crimping channel 32. This channel bracket 38 is positioned just distally of upstanding tabs 44, 46. It will be appreciated that the crimping channel 32 forms with channel bracket 38, a rectangular slidable housing surrounding the jaws 24 of jaw blade 26. Moreover, since the jaw members 24 are formed of outwardly tapered side walls 50, 52, movement of the crimping channel 32 in the distal direction will cause inward movement of the jaw members, while movement of the crimping channel in the proximal direction will result in corresponding proximal movement of channel bracket 38 thereby relieving the jaw members 24 of the crimping forces and permitting the jaw members to open.

Referring now to Figs. 2 and 15, jaw members 24 include generally longitudinal grooves 54, 56 dimensioned to receive a clip 22 therebetween for clipping a body portion. Tissue stop plate 60 shown in Fig. 2, is positioned between jaw blade 26 and crimping channel 32 and includes aperture 62 at the proximal end portion for reception of an appropriate pin (not shown) which extends through the jaw blade 26 and tissue stop plate 60 to maintain alignment of the jaw blade 26 and the tissue stop plate 60 when these components are welded together. At the distal portion of the tissue stop plate a tab 64 is oriented at approximately the same downward angle as the jaws 24 for alignment therewith and includes an arcuate cut-out portion as shown, dimensioned to snugly receive an artery for locating and positioning the artery in the precise area within the jaw blades as required for applying a clip to the artery with predetermined precision. The tissue stop plate is preferably fabricated of a thin stainless steel sheet material.

Referring further to Fig. 2, cover plate 66 is appropriately dimensioned to rest atop the clip clamping mechanism described hereinabove, and supports the row of clips 22. Proximally of clips 22 is positioned a clip follower 68 which is "U" shaped at the distal end to snugly engage and advance the clips under the action of clip feed spring 72 connected thereto at the distal end and to a pin 74 at the proximal end. Pin 74 is in turn connected to cover plate anchor tab 66a while clip pusher bar 78 is positioned for slidable movement thereon between a proximal position and a distal-most portion. When the next clip 22 is engaged by the distal nose 80 of clip pusher 78, distal movement of the clip pusher 78 advances the clip into the slots 54, 56 of jaws 24 of the jaw blade 26.

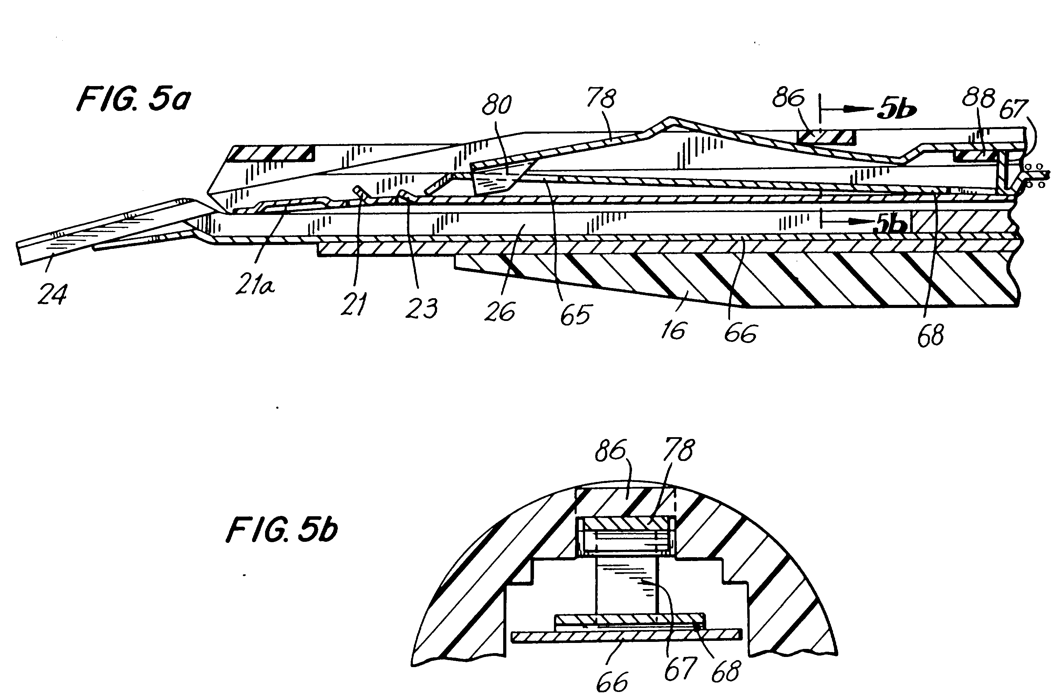

Referring again to Fig. 2, upper housing half section 15 includes a longitudinal slot 82 having bridge connections 84, 86, 88 as shown. In position, the clip pusher bar 78 is snaked over bridge 88 and under distal bridges 86 and 84 such that bridge 88 will act as a stop mechanism to prevent the advancement of clip follower 68 when upstanding tab 67 engages bridge 88 as shown in Fig. 5a. This occurs when the last clip 22 has been advanced and crimped thereby permitting the clip follower to advance to its distal-most position under action of spring 72.

Thus by sliding clip pusher bar 78 between the proximal and distal positions, the clip pusher bar may be alternately positioned with nose 80 behind each successive clip, and thereafter advancing the clip into the jaws 24 of jaw blade 26 by a pusher mechanism in handle section 12 which will be described. The connection between the mechanism in the handle 12 is made with the proximal end portion 90 of clip pusher 78 which extends into the handle section. Further, the connection between the appropriate link of handle 12 with the crimping mechanism of jaw blades 24 is made with the proximal end portion 92 of crimping channel 32 as will be described. The precise action of the handle 12 and its inner mechanism is such that proximal force applied to trigger 16 causes clip pusher 78 to push the next clip 22 into the jaws 24 while simultaneously releasing the crimping channel 32 to the "ready" position for crimping the clip. Next, the operator squeezes handle 18 toward hand grip 20 which causes crimping channel 32 to move distally to crimp the clip positioned within jaws 24, while simultaneously moving clip pusher 78 proximally in position to push the next clip 22 into the jaws 24. These movements are alternately repeated until the last clip 22 is spent.

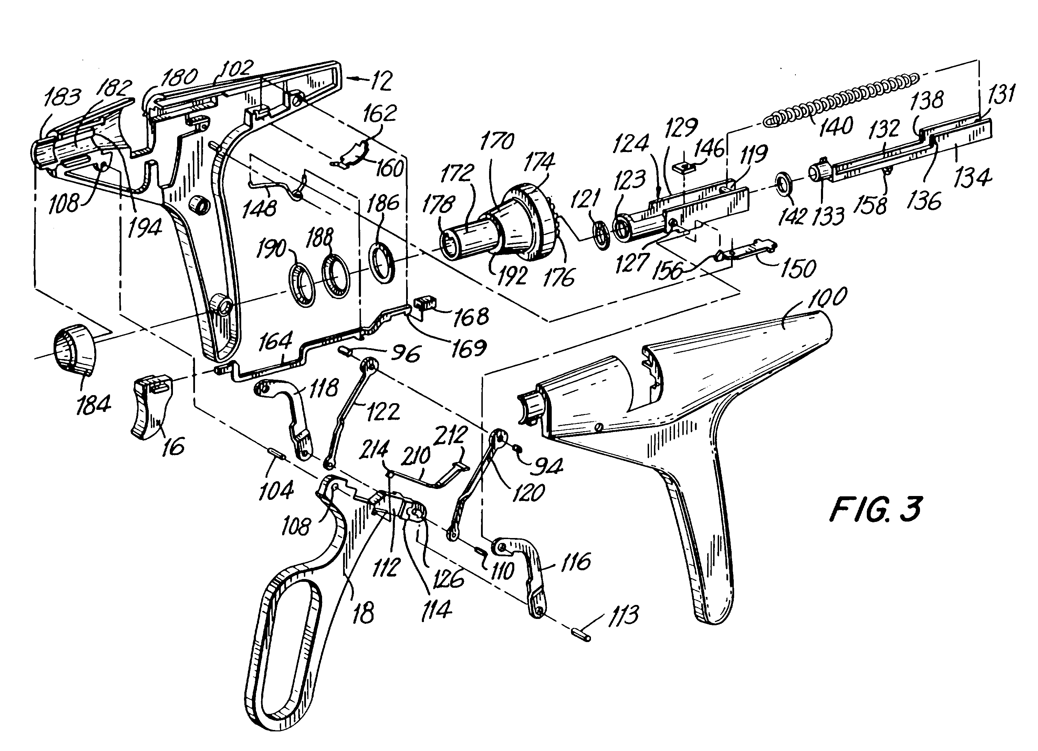

Referring to Fig. 3 the handle section 12 of the apparatus is illustrated with the transmission mechanism for manually activating the endoscopic section described previously, i.e., advancing clips distally and crimping the clips about an artery. The parts of the handle section 12 are separated for convenience of illustration. The handle section 12 includes left body 100 and right body 102. The body parts are fastened together by fasteners such as screws or rivets extending through appropriate bosses. Alternatively, the body parts may be ultrasonically welded or adhesively attached together along their seams or by bosses and transverse rods or pins in engaged relation. The body parts 100, 102 are preferably fabricated of a hard plastic material such as LEXAN Brand polycarbonate material marketed by General Electric Co. Other rigid materials are contemplated. Materials capable of being molded into shape while being able to sustain the forces applied by the transmission mechanism are preferred.

The clip loading and crimping system is divided into two separate systems as described in connection with the endoscopic section. As noted, a first system pushes the clip next in line from a row of clips to a position within a pair of clamping jaws 24 as described in connection with the endoscopic section of the apparatus. The second system closes the pair of jaws 24 around the clip to cause the clip to grip the intended artery, tissue, or other blood vessel, while simultaneously repositioning the clip pusher mechanism to push the clip next in line into position between the jaws. This procedure is repeated alternately and sequentially until all clips are spent.

Referring now to Figs. 16-19, in conjunction with Fig. 3, the clip pusher and clamping loading mechanism will now be described. Handle 18 is pivotally mounted via aperture 108 on pin 104 extending transversely of the body parts. The handle 18 includes a rearward extension 112 which defines arcuate slot 126 through which pin 110 extends. Pin 113 extends through aperture 114 and functions as a pivot for left channel link 116 and right channel link 118 which extend in a generally forward direction. Rearwardly directed left pusher link 120 and right pusher link 122 are mounted for pivotal motion on pin 110 extending through arcuate slot 126.

At the opposite ends left channel link 116 and right channel link 118 are pivotally mounted to channel tube 124 by pivot pins 127, 129 formed integral therewith and pusher links 120, 122 are connected to transverse pins 94, 96 arranged for slidable movement within the forward cut-out portion 132 of pusher tube 134 for engagement with shoulders 136, 138 of the pusher tube when the links are moved in the proximal direction. Main spring 140 connects channel tube 124 with pusher tube 134 via pins 119, 131, such that the spring is loaded when the tubes are separated by squeezing handle 18 toward handle grip 20 causing distal movement of channel tube 124 and proximal movement of pusher tube 134.

Referring now once again to Figs. 3 and 16 in conjunction with Fig. 2, it can be seen that pusher tube 134 is connected to clip pusher bar 78 by proximal end tabs 90 which are inserted by squeeze and release action into the distal opening 133 of pusher tube 134 with annular steel pad 142 positioned as an interface between the plastic pusher tube and the steel pusher bar. Similarly, the crimping channel 32 is connected to the channel tube 124 by insertion of the proximal legs 92 into the distal opening 123 of channel tube 124 with annular steel pad 121 positioned as an interface between the plastic channel tube 124 and the steel legs 92. With the connections described, the crimping channel and clip pusher are free to rotate independently of the channel tube and pusher tube as permitted by the rotation of the proximal legs 90 and 92, within the distal opening 133 of pusher tube 134 and opening 123 of channel tube 124.

Referring once again to Fig. 3, latch plate 150 is pivotally mounted and biased upward toward apertured plate 146 in lower wall of channel tube 124 by spring 148 such that tongue 156 enters the aperture of plate 146 when the channel tube 124 is moved to its proximal position. This prevents unwanted forward movement of the channel tube 124 prior to advancing a clip in position within jaw members 24 of jaw plate 26. Release of tongue 156 is accomplished by engagement of the latch plate 150 by pin 158 extending downwardly from pusher tube 134 when pusher tube moves distally under action of mainspring 140 as will be developed further. Similarly, pusher release leaf spring 160 is positioned for entry of tab 162 into a slot 161 in the bottom wall of pusher tube 134 when the tube is moved proximally by pusher links 120, 122 against the force of mainspring 140, permitting the leaf spring 160 to retain the pusher tube in position against the force of the mainspring 140. Release of the pusher release leaf spring 160 is accomplished by proximal movement of release lever 164 via finger activated pusher release button 16 supported at the proximal end by lever support block 168 which slidably moves against the lower wall of pusher tube 134.

In operation, squeezing the handle 18 toward hand grip 20 causes pusher links 120, 122 to pivot and move proximally, resulting in proximal movement of pusher tube 134 by engagement of pins 94, 96 with shoulders 136, 138 of pusher tube 134. Proximal movement of pusher tube 134 continues with pusher release spring 160 continuously biased upwardly until tab 162 enters the slot 161 in the bottom wall of pusher tube 134 thereby retaining pusher tube 134 in position against the bias of mainspring 140. Simultaneously, this action withdraws clip pusher 78 to a position just proximal of the next clip 22 in preparation for pushing the clip distally between the jaw members 24 of jaw blade 26. Retention of the pusher tube in this proximal position by release spring 160 also retains the clip pusher 78 in the corresponding position until the clip next in line is to be pushed into the jaws 24. When this is desired, proximal movement of pusher release button 16 causes proximal movement of release lever 164 and engagement of proximal tip 169 with pusher release spring 160 causing downward movement of the spring and corresponding release of the pusher tube 134. This action causes distal movement of pusher tube 134 and clip pusher 78 with corresponding distal engagement of nose 80 with the next clip 22 thereby positioning the clip into the slots 54, 56 of jaws 24.

Once clip 22 is positioned within jaws 24 of jaw blade 26 squeezing handle 18 proximally toward hand grip 20 causes distal and pivotal movement of channel links 116, 118, resulting in distal movement of channel tube 124 and corresponding distal movement of crimping channel 32. This action causes channel bracket 38 together with crimping channel 32 to engage and squeeze the jaw members 24 of jaw blade 26 thereby crimping the clip 22 positioned therebetween. At the same time, the proximal movement of pusher tube 134 resets clip pusher 78 to a position just proximal of the next clip in readiness for the next clipping operation. Reentry of the tab 162 of pusher release spring 160 into slot 161 of pusher tube 134 retains the pusher 78 in position behind the next clip 22.

Referring further to Fig. 3 in conjunction with Fig. 2, the feature relating to the rotatable endoscopic section will be described. Rotating collar 170 is constructed of the same material as the handle, i.e. preferably a polycarbonate material such as LEXAN brand material. This collar 170 includes a distal cylindrical nose section 172 and a proximal barrel section 174. The proximal face of the barrel section 174 includes a plurality of proximally extending teeth 176 positioned circumfer-entially about the proximal face of the barrel section and the cylindrical nose section includes an inwardly extending rib 178 at the distal end. In the assembled condition, the cylindrical nose section rests within the cylindrical distal opening 182 of the distal end of the handle and nose piece 184 is fitted over the distal cylindrical end 183 of the handle as shown in Figs. 16-18. Bearing washer 186 and spring washers 188, 190 are positioned between shoulder 192 of collar 170 and shoulder 194 formed in the handle body to bias the rotatable collar in the proximal direction causing tooth 180 on the handle body to engage the teeth 176 of the collar 170 to thereby fix the rotatable orientation of the collar. When the surgeon desires to change the angular orientation of the endoscopic section, the collar 170 is merely pushed distally to disengage tooth 180 to free the collar and permit rotation relative to the handle body. Such rotation of the collar is clearly permitted by the fact that the cylindrical nose section of the collar is fit snugly within the corresponding cylindrical distal section 182 of the handle. Except when the tooth 180 of the handle body is engaged with teeth 176 of collar 170, the collar is otherwise free to rotate within the handle.

Referring now to Fig. 2 in conjunction with Figs. 1 and 3, the distal cylindrical section 172 of collar 170 includes a distal cylindrical opening dimensioned to receive the endoscopic cartridge formed of upper half 15 and lower half 16, with distally positioned tooth 178 of collar 170 positioned within longitudinally extending groove of upper cartridge half 15 to cause the cartridge to rotate with the collar 170. Similarly, the proximal legs 90 of clip pusher bar 78 are permitted to rotate within the distal end portion 133 of pusher tube 134 and the proximal legs 92 of the crimping channel 32 are permitted to rotate within the distal end portion 123 of channel tube 124. Thus, the entire endoscopic section may be selectively rotated by the surgeon by simply pushing collar 170 in the distal direction sufficient to disengage tooth 180 on the handle body and by rotating the collar 170 until the endoscopic section reaches the desired angular orientation. Thereafter, by merely releasing the collar the bias of spring washers 190, 188, causes the collar to move proximally, such that tooth 180 on the handle body engages the appropriate teeth 176 on the collar 170 to lock the position of the collar and the endoscopic section. This feature represents a significant advance in endoscopic surgery when it is fully appreciated that the orientation of human tissue or arteries to be clamped vary widely and that selectivity of orientation of the clip is a necessity. Without the above-described feature, the entire apparatus must otherwise be rotated until the proper orientation of the endoscopic section is reached. Such rotation of the entire apparatus during a delicate surgical operation would be prohibitive.

Referring now to Figs. 12, 13, 14 and 15, the jaws of the clamping section of the apparatus are illustrated. Fig. 12 illustrates the jaws 24 of the apparatus in position after having applied a clip 22 about an artery 98 or other blood vessel to stop the blood flow as illustrated graphically in Fig. 15. As shown in Fig. 15, the jaw members 24 include longitudinally extending grooves 54, 56 which receive clip 22 as the clip is advanced distally by pusher bar 78. It can be seen that at the time the jaw members 24 are clamped together, the nose 80 of pusher bar 78 has been withdrawn proximally to a position proximal of the next clip 22 and is not permitted to advance in the distal direction until the surgeon pulls pusher release button 16 in the proximal direction to release the clip pusher mechanism described previously. Tab 23 prevents the next clip from moving proximally with the pusher bar when the pusher bar returns to a position proximal of the next clip for the sequence. Also, prior to release of the pusher bar for distal movement, fingers 21 upstanding from track 66 prevent distal movement of the next clip preventing the clip from falling out through the jaws. In addition, it is significant to note that once the jaw members 24 are released from their clamped condition shown in Fig. 12, by release of handle 18, clamping of the jaw members 24 may not be repeated until the pusher release button 16 has been depressed to deliver the next clip between the jaws 24. Such clamping action is prevented by the position of tongue 156 within the aperture of plate 146 in the bottom wall of channel tube 124 under the upward bias of spring 148. This position prevents distal movement of the channel tube 124 until the tongue 156 is released from the aperture of plate 146 by engagement of downwardly extending finger 158 of pusher tube 134 with latch plate 150 when pusher tube 134 is caused to advance distally by releasing pusher release button 16.

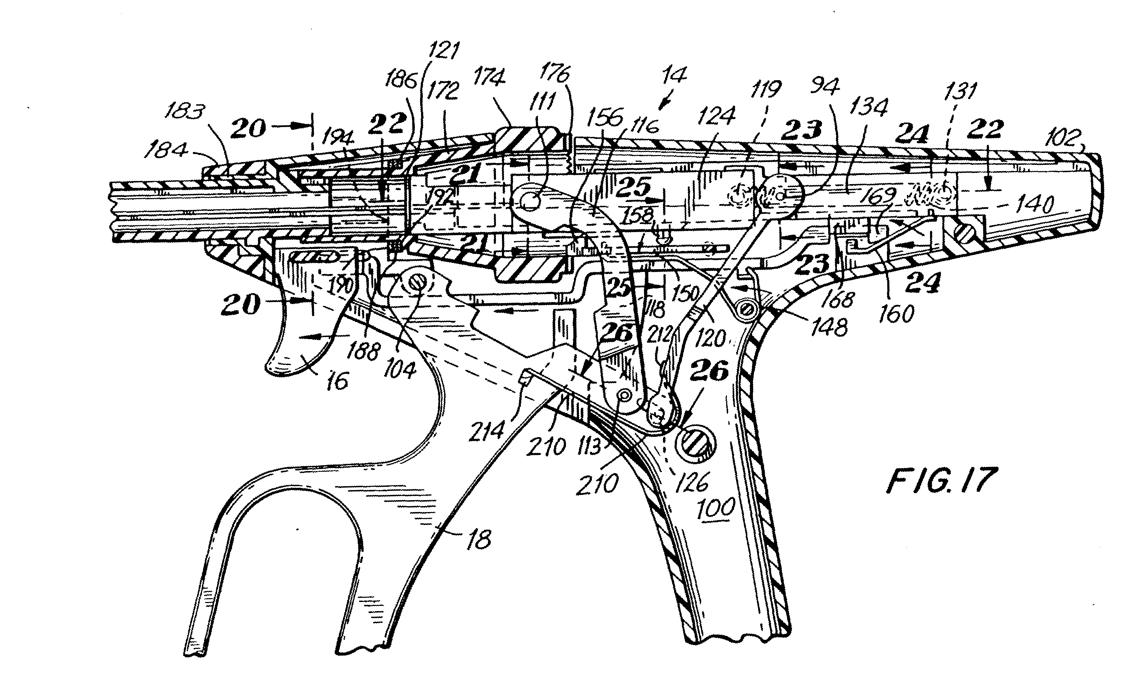

The release action on tongue 156 is shown more clearly in Fig. 17 which illustrates the handle with the pusher tube 134 in the distal-most position after pusher release button 16 has been depressed to advance the next clip into the jaw members 24 of jaw blade 26. It can be seen clearly in Fig. 17 that finger 158 has engaged latch plate 150 pivoting the latch plate downwardly in the counter clockwise direction against the upward bias of latch spring 148. It will similarly be appreciated that the proximal movement of pusher tube 134 during the squeezing action of handle 18 and jaw members 24 will continue until the tab 162 of the upwardly biased pusher release spring 160 engages the slot 161 in the bottom wall of the proximal section of pusher tube 134 thereby causing the pusher tube to be locked in position corresponding to the nose 80 of pusher bar 78 being positioned just proximal of the next clip 22 for the next clip advancing step as described hereinabove. It can be appreciated readily that this safety feature avoids the possibility of squeezing the jaw members 24 about an artery or other tissue with no clip positioned therebetween. Thus, the only time in the sequence of operation that the jaws can be squeezed is after the advancement of a clip 22 therebetween.

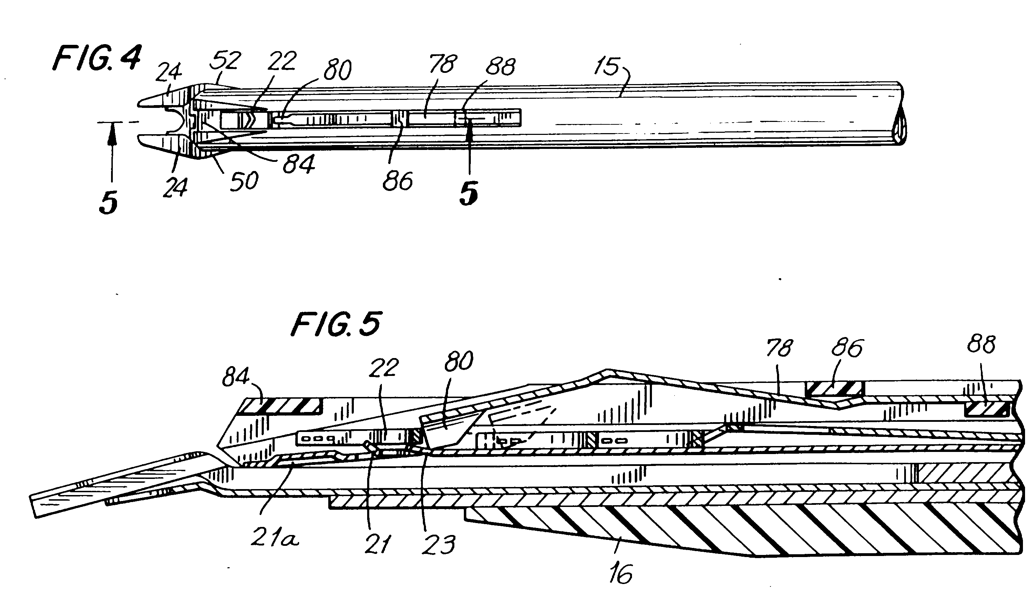

Referring now to Figs. 4-11, the inner mechanism and function of the distal portion of the endoscopic section are illustrated. In Fig. 4 a plan view from above, is shown of the distal portion of the endoscopic section, illustrating the jaw members 24 and the nose 80 of pusher bar 78 in position to advance the clip 22 into the jaw members. At this time, the row of clips 22 are advanced to their distal-most positions under bias action of clip feed spring 72 between anchor shaft 74 on cover plate pin anchor tab 66a and pin 71 on clip follower 68 shown in Fig. 2. Fig. 5 is a cross-sectional view taken along lines 5-5 of Fig. 4 illustrating the clip 22 and the nose 80 of pusher bar 78 in position just proximally thereof. The view of the nose 80 of pusher bar 78 shown in dotted lines is intended to illustrate the proximal-most position of the nose 80 of pusher bar 78 as represented by the last portion of the squeezing motion of handle 18 toward hand grip 20 thus establishing with certainty, that the nose 80 of clip pusher 78 is in fact positioned proximally of the next clip 22 after the handle 18 is released and the nose 80 of pusher bar 78 is permitted to move distally a small distance as shown behind clip 22 as represented by relaxation of the combined tolerance build-up of the components interacting with each other. Escapement means in the form of upstanding tabs 21 in cover plate 66 prevent the next clip 22 from distal movement before it has been advanced distally by the pusher bar 78. Arch 21a assists proper orientation of the clip entering the jaws. Tab 23 prevents proximal movement of clip 22 once it has been advanced distally by nose 80, i.e. the proximal return movement of nose 80 does not move clip proximally (by friction) along with the nose.

Fig. 5a illustrates still another significant feature of the present invention which prevents further distal advancement of the clip pusher 78 after the last clip 22 has been advanced distally into the jaws 24 and clamped about an artery. In particular, the proximal portion of clip follower 68 includes upstanding tab 67 which is positioned and dimensioned to engage bridge 88 on upper cartridge half 15 when clip follower 68 assumes the distal-most position shown in Fig. 5a under bias of spring 72. This position is assumed by clip follower 68 after the last clip has been advanced distally into the jaws 24. Thus, the engagement of upstanding tab 67 with bridge 88 prevents further distal movement of the clip follower at this stage. Furthermore, as shown in Fig. 5a, the distal position of clip follower 68 results in slot 65 now assuming 1B distal-most position such that nose 80 of clip pusher bar 78 may drop into slot 65 thus preventing further distal movement of the pusher bar 78 after the last clip has been spent. This is a further safety feature of the invention in that the apparatus is inactivated after the last clip is spent, thus avoiding the possibility of the surgeon clamping the jaws 24 about an artery with no clip in position. Fig. 5b is a cross-sectional view taken along lines 5a-5a of Fig. 5 illustrating the clip follower 68 and the clip cover plate 66 in the position shown in Fig. 5a.

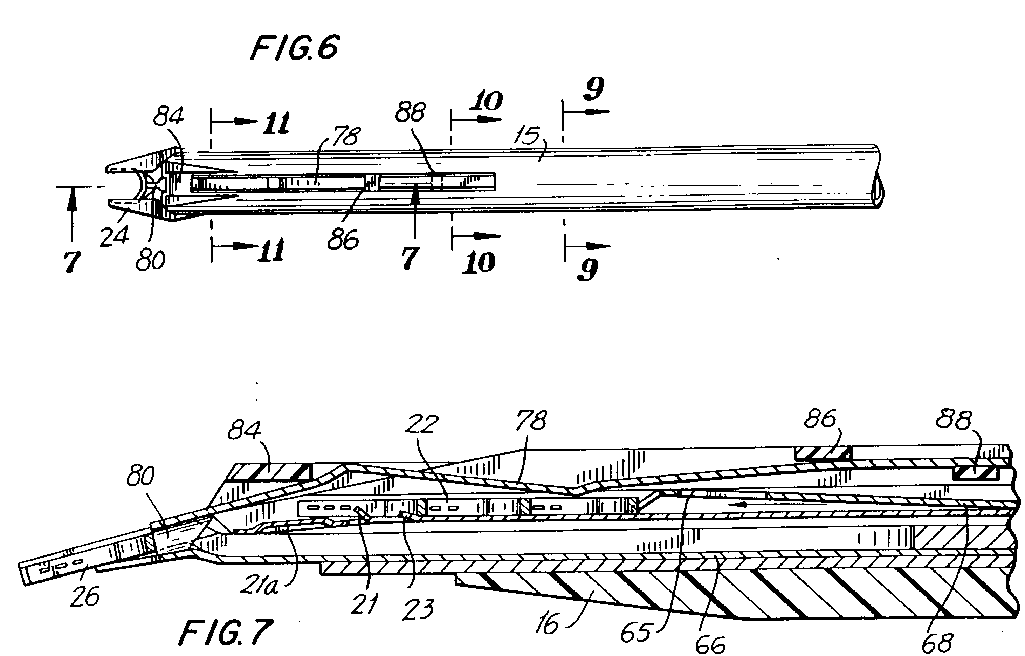

Referring now to Fig. 6, a plan view from above similar to Fig. 4 is shown of the distal portion of the endoscopic section with clip 22 shown in Fig. 5 now advanced distally to a position within the jaws 24 by nose 80 of clip pusher 78. Fig. 7 is a cross-sectional view taken along lines 7-7 of Fig. 6 illustrating the clip 22 and clip pusher bar 78 in the distally advanced position after advancing clip 22 into the jaws 24.

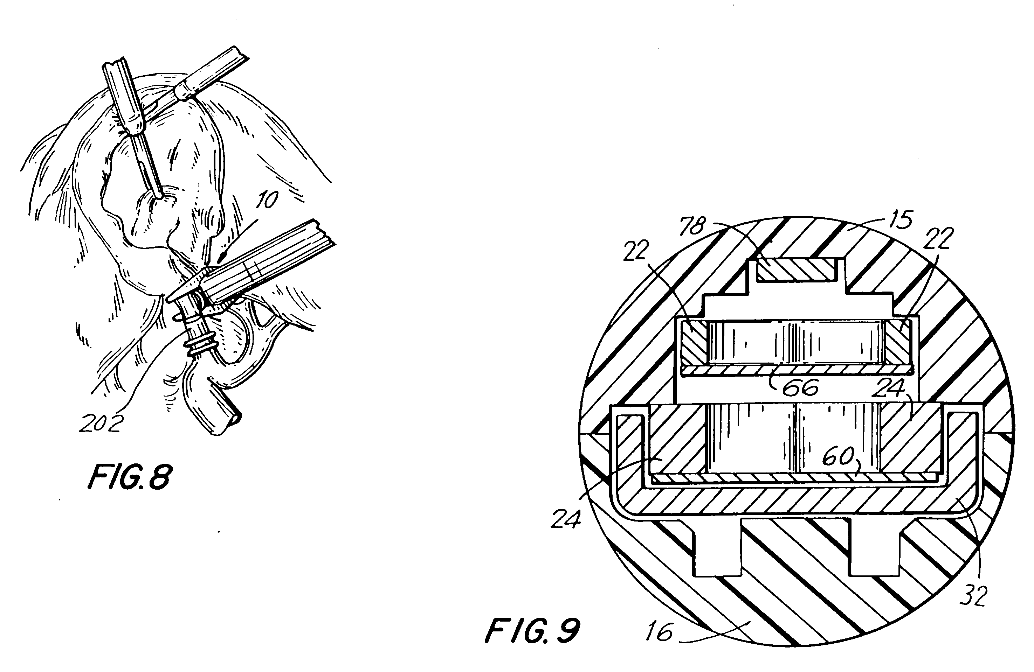

Fig. 8 is a schematic view of the distal end of the completely disposable instrument of the present invention positioned in the body cavity. The jaws of the intrument are positioned around cystic duct 202 where two clips have already been closed and a third is being positioned for closure.

Fig. 9 is a cross-sectional view taken along lines 9-9 of Fig. 6, illustrating the crimping channel 32, the tissue stop 64, clip 22, pusher bar 78 and jaws 24, and cover plate (or clip track) 66.

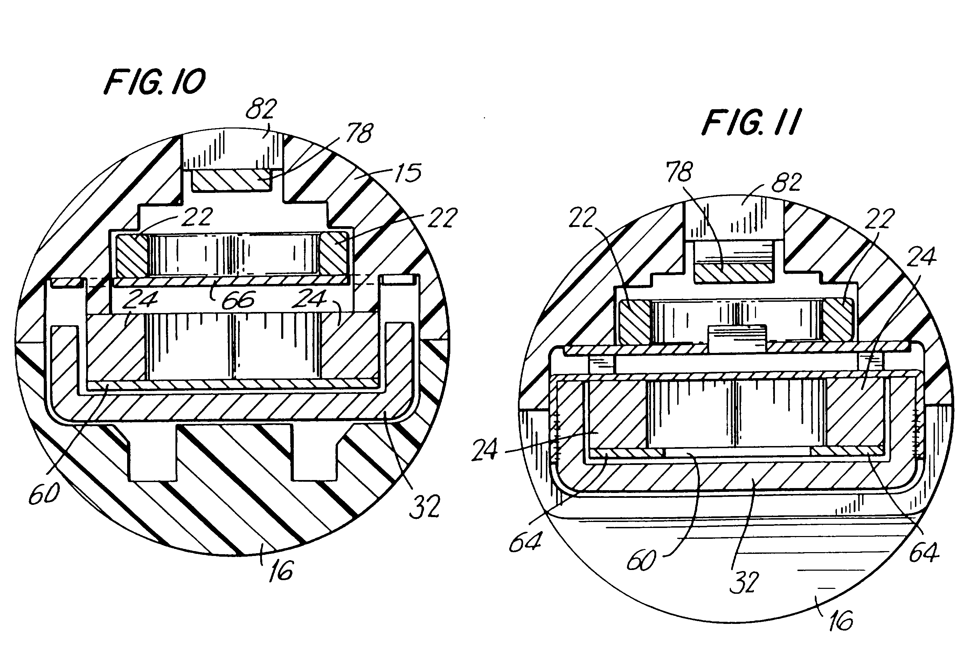

Fig. 10 is a similar cross-sectional view taken along lines 10-10 of Fig. 6 illustrating the clip advancing components. Fig. 11 is a cross-sectional view similar to Fig. 10 illustrating the clip advancing mechanism distal of the cross-section shown in Fig. 10.

Referring now to Figs. 16-19, the inner clip advancing and jaw squeezing mechanism is shown in various stages of the operation. Fig. 16 is an elevational cross-sectional view of the handle 18 of the apparatus, illustrating the pusher tube 134 in the proximal-most position corresponding to the position of the pusher bar 78 shown in Fig. 5, i.e. with the nose 80 just proximal of the next clip 22 in readiness to activate the clip distally into the jaws 24. Additionally, with pusher tube in the proximal position, downwardly extending finger 158 has moved out of engagement with latch 150 thereby permitting tongue 156 to enter the aperture of channel latch plate 146 thus preventing any distal movement of channel tube 124. This condition locks handle 18 in the distal position whereby squeezing the handle toward hand grip 20 is prevented.

Referring now to Fig. 17, there is shown a cross-sectional view of the handle 18 of the apparatus with the pusher tube in the distal-most position corresponding to the position of pusher bar 78 as shown in Fig. 7, i.e. with the clip 22 advanced distally into the jaws 24 of jaw blade 26. As can be seen further in Fig. 17, the distal position of pusher tube 134 has now resulted in release of tongue 156 of latch plate 150 from the aperture of channel latch plate 146 in the bottom wall of channel tube 124 thereby permitting advancement of channel tube 124 and crimping channel 32 distally to squeeze jaws 24 in conjunction with channel bracket 38.

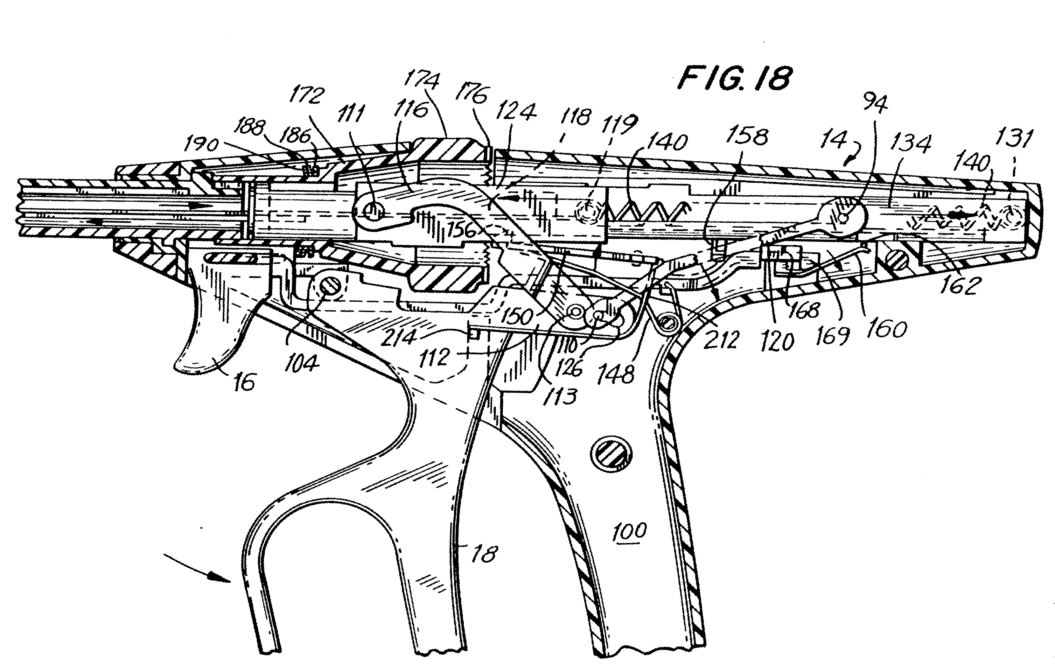

Referring now to Fig. 18, a cross-sectional view of the handle 18 is shown after the crimping action has taken place on clip 22 positioned within jaws 24 shown in Figs. 16 and 7. The position of the components shown in Fig. 18 corresponds to the position of the jaws shown in Figs. 12-15, i.e., in the clamped position about clip 22. In the cross-section shown in Fig. 18, the pusher tube 134 in the proximal-most position and the channel tube is in the distal-most position such that crimping channel 32 and channel bracket 40 are in the distal-most position shown in Figs. 12-15.

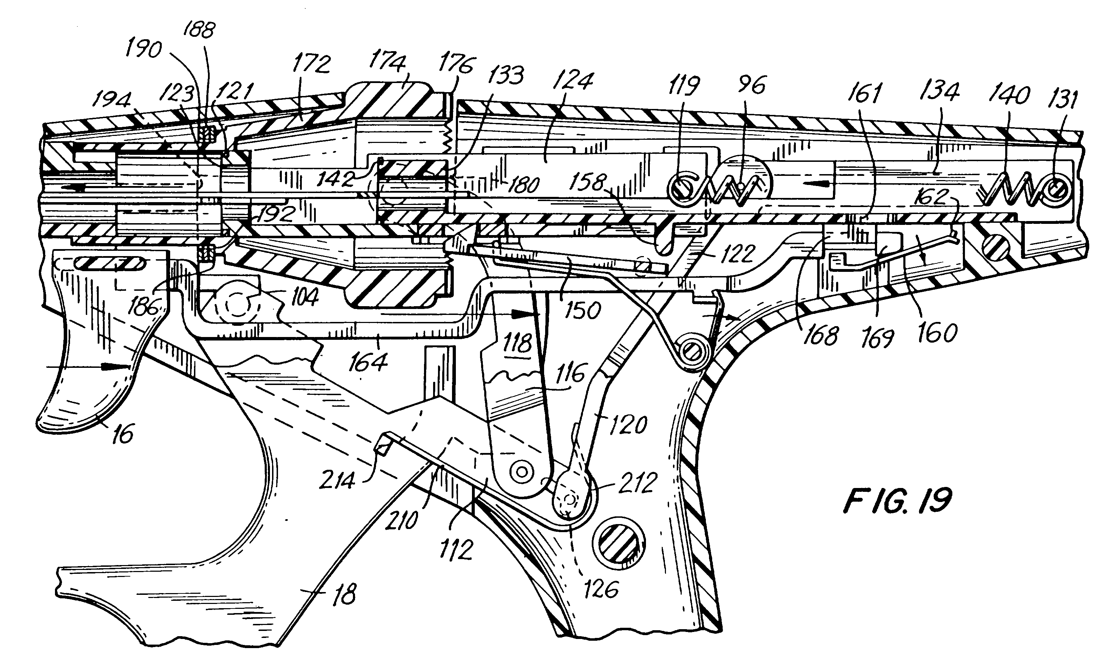

Referring to Fig. 19 a cross-sectional view of the handle 18 is shown after the last clip 22 has been spent, i.e. corresponding to the position of the clip follower 68 shown in Fig. 5a. As noted hereinabove, the clip follower 68 of the endoscopic section is prevented from moving further distally by interaction with bridge 88 formed in upper cartridge half section 15. Additionally as noted, it can be seen in Fig. 5a that clip follower 68 defines slot 65 at the distal portion which is bounded on the distal end by a bridge 63 which is positioned to engage the nose 80 of pusher bar 78 when clip follower 68 has advanced to the distal-most position shown in Fig. 5a, i.e. after the last clip has been spent. In this position, the clip follower is now sufficiently distal to engage the nose 80 of clip pusher bar 78 which is biased downwardly by the configuration of pusher bar 78 and by the resilient properties of the material from which the pusher bar is fabricated, i.e. stainless steel. This engagement with bridge 63 prevents further distal movement of clip pusher bar 78 and correspondingly of pusher tube 134. By preventing pusher tube 134 from distal movement with channel tube 124 locked in its proximal position by tongue 156 of latch plate 150, further squeezing action of handle 18 toward hand grip 20 is also prevented. This locking action correspondingly prevents distal movement of crimping channel 32. As shown in Fig. 19, pusher release button is depressed but full distal movement of pusher tube 134 is prevented by the engagement of nose 80 of pusher bar 78 with bridge 63 of clip follower. Only a small distal movement is permitted as seen by the position of slot 161 in pusher tube 134 relative to the position of pusher release spring 160. This locked position of pusher tube 134 also serves to prevent downwardly depending finger 158 of pusher tube 134 from distal movement sufficient to release tongue 156 of latch plate 150 from channel tube 124 as shown. Thus, the crimping mechanism is inactivated for safety purposes.

This feature is extremely significant in disabling the apparatus from squeezing jaws 24 of the jaw blade 26 on an artery alone, i.e. with no clip positioned therebetween. Further, all movement of the clip advance mechanism is now prevented after the last clip has been spent. At this stage, the entire instrument is considered disposable and may be disposed of in accordance with correct approved disposal procedures.

Referring once again to Fig. 3 in conjunction with Figs. 16-19, the lost motion spring 210 is shown having transverse arms 212 and tab 214. Spring 210 provides bias force on pusher links 120, 122 such that squeezing action on handle 18 maximizes proximal movement of pusher tube 134. Thus, partially closing the jaws 24 of jaw blade 26 will cause pusher tube 134 to move sufficiently proximal to make certain that pusher bar 78 has moved proximally of the next clip 22. Without such movement it may be possible for the surgeon to squeeze the jaws, not fully appreciating that the pusher bar 78 has not moved to a position proximal of the next clip 22. This proximal movement of the pusher bar transmission is thus assisted by lost motion spring 210 which maximizes the repositioning movement of the pusher bar 78 behind the next clip whether the jaws are squeezed fully or partially. In particular, the proximal bias provided by spring 210 on pusher links 120, 122 maximizes the movement of pusher tube 134 in relation to the movement of handle 18 by maintaining pusher links 120, 122 in their proximal-most positions prior to squeezing the handle 18. This maximum proximal movement of pusher links 120, 122 in turn results in proximal movement of pusher tube sufficient to engage tongue 162 of release spring 160 thus making certain that pusher bar 178 is repositioned sufficiently proximally to advance the next clip 22 into the jaw members 24.

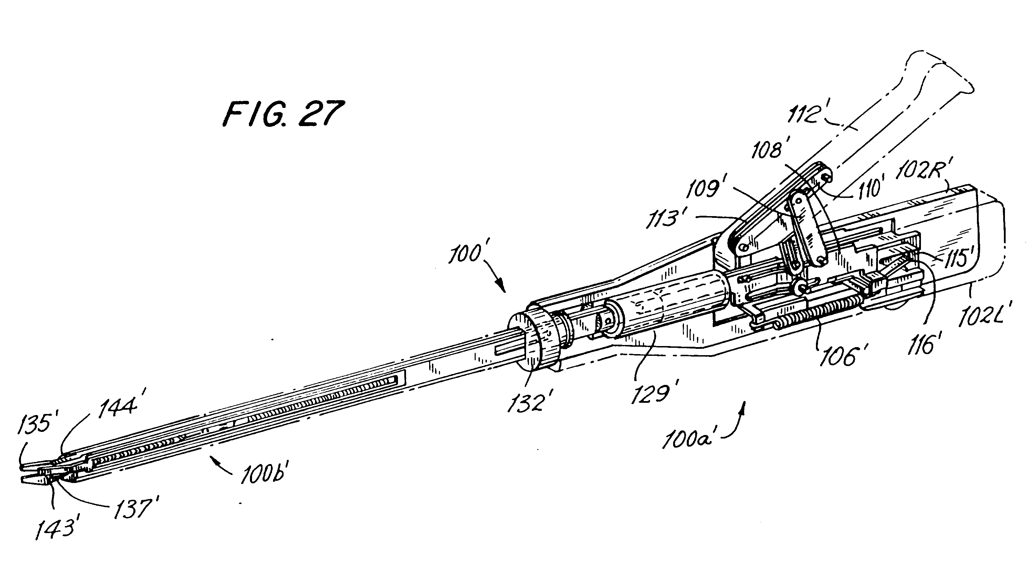

Fig. 27 shows a cutaway perspective view of a second embodiment of the present invention 100' which generally comprises an actuating body 100a' supporting a non-detachable endoscopic portion 100b'. Included are means for actuating the instrument, transmission means, means for applying a surgical clip to a blood vessel or the like, means for locking the instrument, and means for unlocking the instrument and repositioning another clip. Clip applier 100' is intended to be fully disposable.

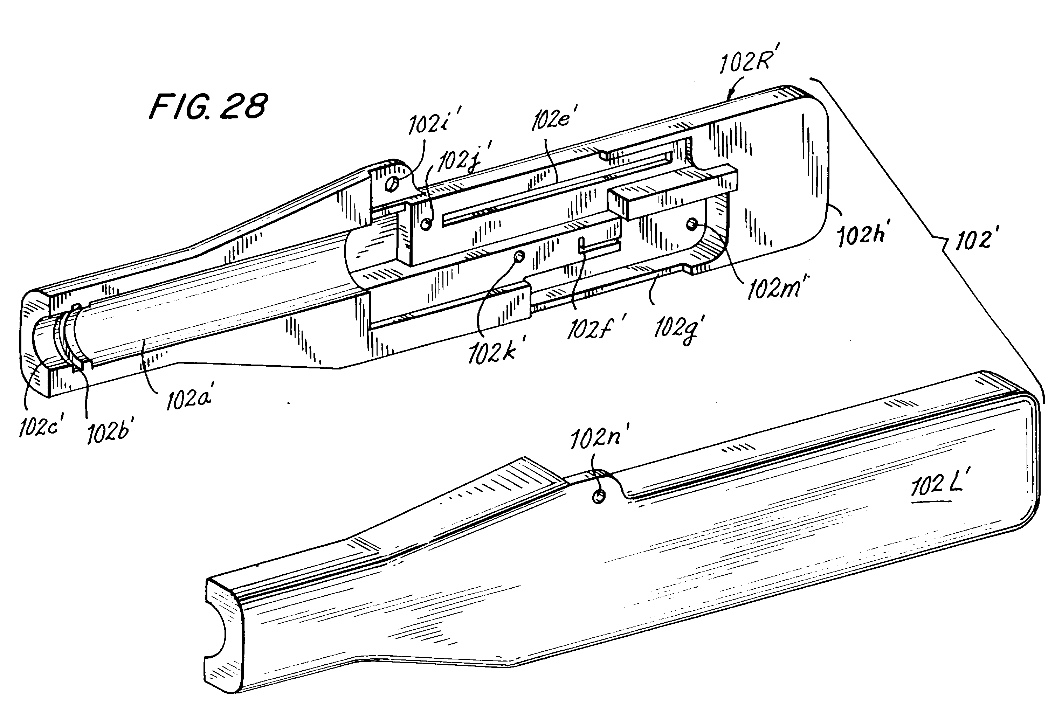

More particularly, referring now to Figs. 27 and 28, frame 102' comprises a left portion 102L' and a right portion 102R'. These portions are optimally fastened together by means of fastening screws, although rivets, welds, adhesives, or other means of joining the frame portions may be used. Frame 102' is elongated and has an interior surface defining a distal opening 102c', a proximal end 102h', an interior distal chamber 102a', a circumferential groove 102b', an upper guideway 102e' to receive pins 107' and 103' (see below), a mounting slot 102f' (to receive spring 116'), elongated access aperture 102g', and pin mounting holes 102i', j', k', m' and n' for receiving pins 101', 119', 105', 118' and 101', respectively. The frame is of overall size and shape convenient for being held in the hand.

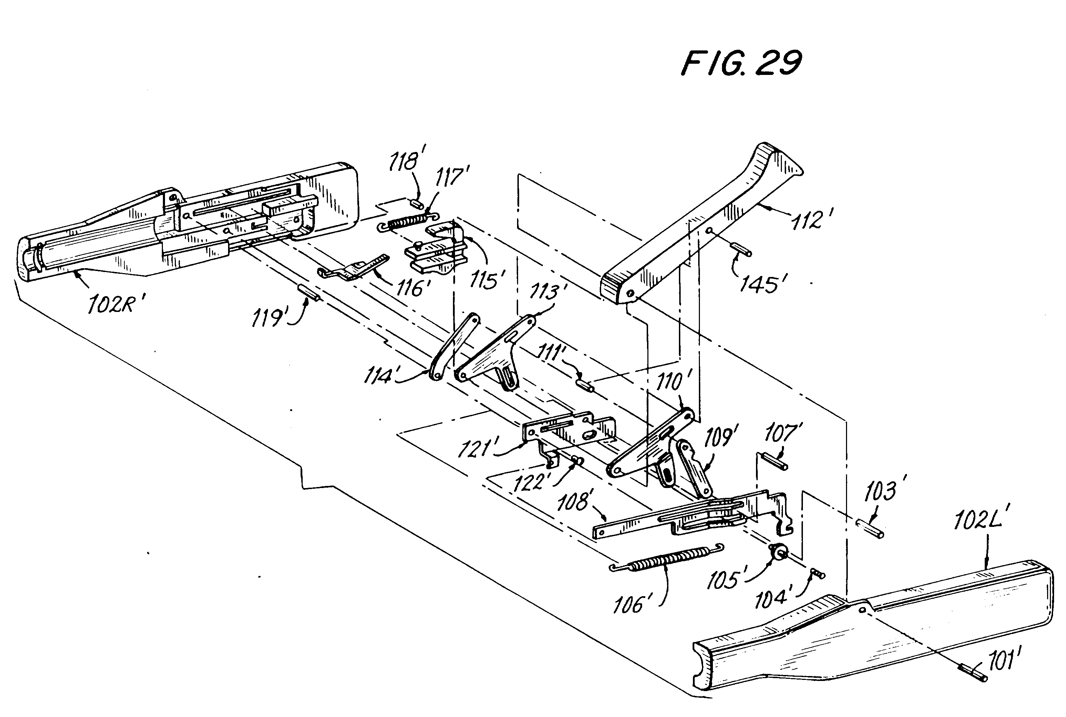

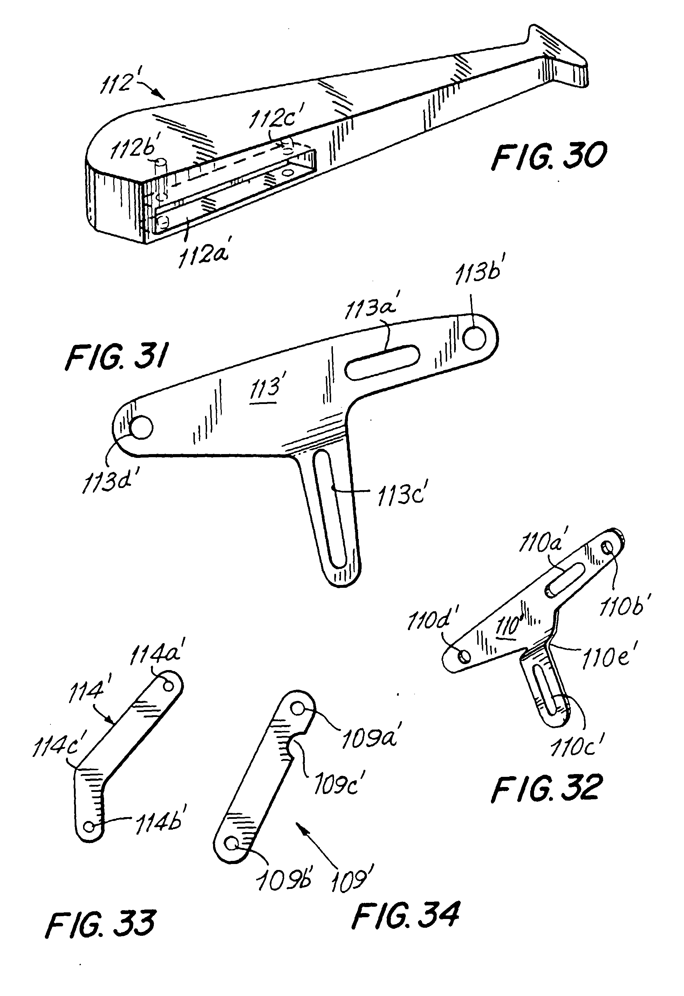

Referring additionally now to Figs. 29, 30, 31, 32, 33 and 34, handle 112' is pivotally mounted to the frame 102' by means of handle pin 101' which is disposed through holes 102n' and 102i' in the frame, and hole 112b' in the distal portion of the handle 112'. Handle 112' also has an elongated cavity 112a' for receiving the toggle levers 110' and 113'. Hole 112c' receives lever pin 145'. Handle 112' serves as a means to activate the instrument when said handle is pivoted clockwise by the user of the instrument.

Toggle levers 113' and 110' are T-shaped levers which are pivotally mounted to the handle 112'. Lever 113' has a proximal aperture 113b' for receiving pin 145', a distal aperture 113d' for receiving pin 101', said pins being respectively disposed through holes 112c' and 112b' in the handle 112'. Lever 113' also has an elongated slot 113a' for receiving pin 111', and a lower slot 113c' for receiving pin 103'.

Toggle lever 110' is similar to toggle lever 113' except that the lower portion is offset to the left by bend 110e'. Slot 110c' in the lower portion is for receiving pin 103'. Slot 110a' in the upper portion receives pin 111', and apertures 110b' and 110d' receive pins 145' and 101', respectively. The lower legs of the toggle levers 113' and 110' transmit motion of handle 112' to the rear channel 121' via pin 103'.

Pusher bar rear link 109' is a flat elongated piece having an aperture 109a' for receiving pin 111', an aperture 109b' for receiving pin 107', and a curved notch 109c' for accommodating pin 145' when the handle 112' is pushed down into a closed position. Pusher bar rear link 109' transfers movement from the handle 112' to the rear pusher bar 108'.

Pusher bar front link 114' is an elongated flat piece having a bend 114c', aperture 114a' for receiving pin 111', and aperture 114b' for receiving pin 119'.

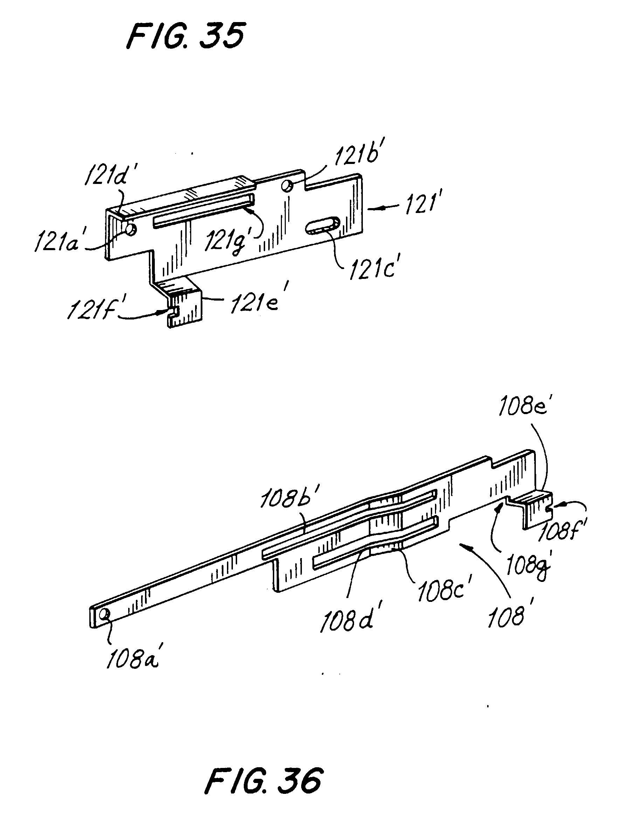

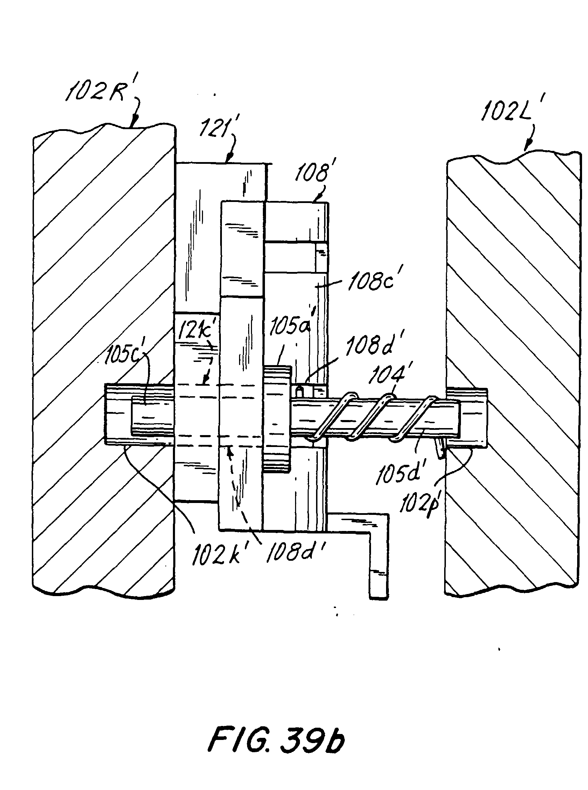

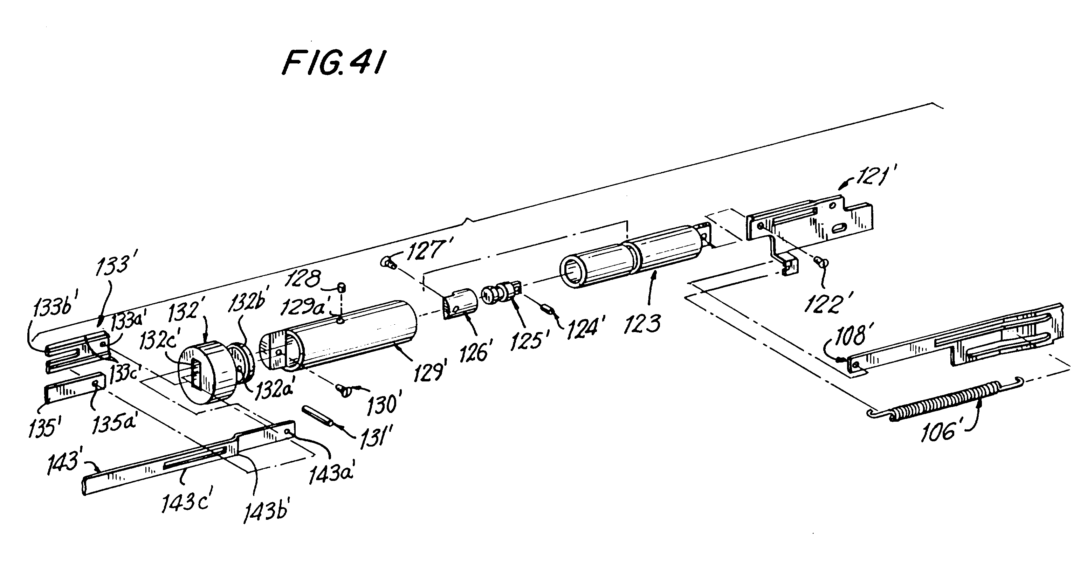

Referring additionally now to Figs. 35 and 36, rear channel 121' is longitudinally movable and has a distal aperture 121a' to receive screw 122' for attachment to the rear channel tube 123' (see Fig. 41.). Rear channel 121' also has a proximal aperture 121b' to receive pin 103'. Slot 121g' receives pin 119' and aperture 121c' receives channel lock pin 105'. Rear channel 121' has an overhang 121d', and a lower offset flap 121e' with notch 121f' for holding the distal end of spring 106'. Rear channel 121' receives pivotal movement from toggle levers 110' and 113', and transfers motion linearly to the rear channel tube 123'.

Rear pusher bar 108' is longitudinally movable and has a distal aperture 108a' to receive pin 124' for mounting the distal end to the rear pusher bar tube 125' (see below). Rear pusher bar 108' also comprises upper slot 108b' for receiving pin 119', lower slot 108d' for receiving the channel lock pin 105', camming surface 108c', and offset flap 108e' with proximal notch 108f' for receiving the proximal end of spring 106'. Rear pusher bar 108' further possesses a stopping edge 108g' which provides a catch means for engaging the locking flap of the pusher bar stop spring 116' (see below). Rear pusher bar 108' provides means for transferring pivotal movement from link 109' linearly to rear pusher bar tube 125'.

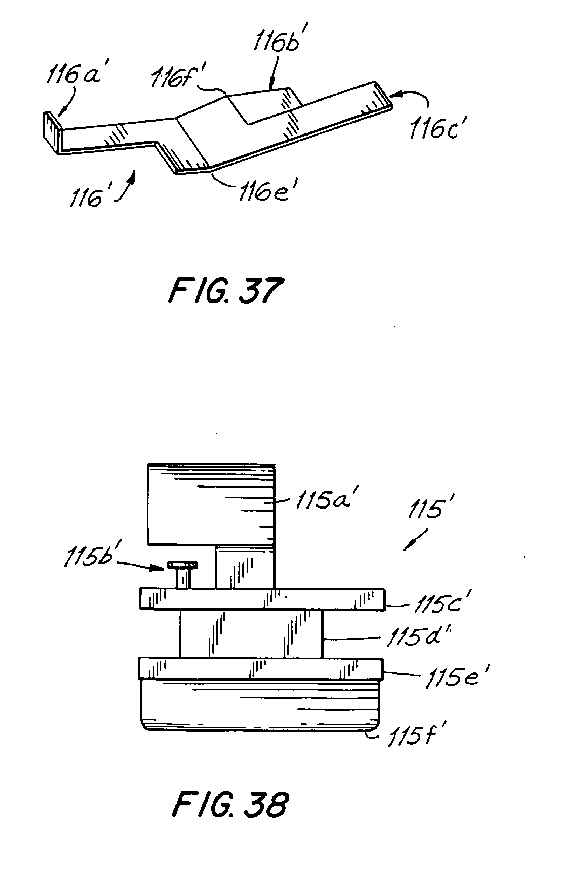

Referring additionally now to Figs. 37 and 38, resilient stop spring 116' has an elongated distal end with flap 116a' for mounting into slot 102f' in the right frame 102R'. Spring 116' has a bend 116e', and a proximal end divided into a pusher bar stop latch 116c' which provides means for locking the rear pusher bar 108' and a camming surface 116b' which is angled by bend 116f', as illustrated. Slide member 115' has a cam member 115a', an anchor post 115b' for engaging the distal hook end of spring 117', an upper base 115c', a connecting portion 115d', a lower base, 115e', and a curved pushing surface 115f'. Slide member 115' rides along the longitudinal access aperture 102g' and is mounted in the frame 102' such that the upper base is in the enclosed interior space of the frame 102', and the lower base 115e' and pushing surface 115f' project outside of the frame. The cam member 115a' is engagable with the camming surface 116b' of the spring 116'. Upon moving distally the cam member 115a' pushes spring 116' downward, thereby pivoting the stop latch 116c' into a non-engagable position below the stopping edge 108g' of the rear pusher 108'.

Spring 117' is connected by a distal hook to the anchor post 115b' and by a proximal hook to the pin 118', which is received into aperture 102m' in right frame portion 102R'.

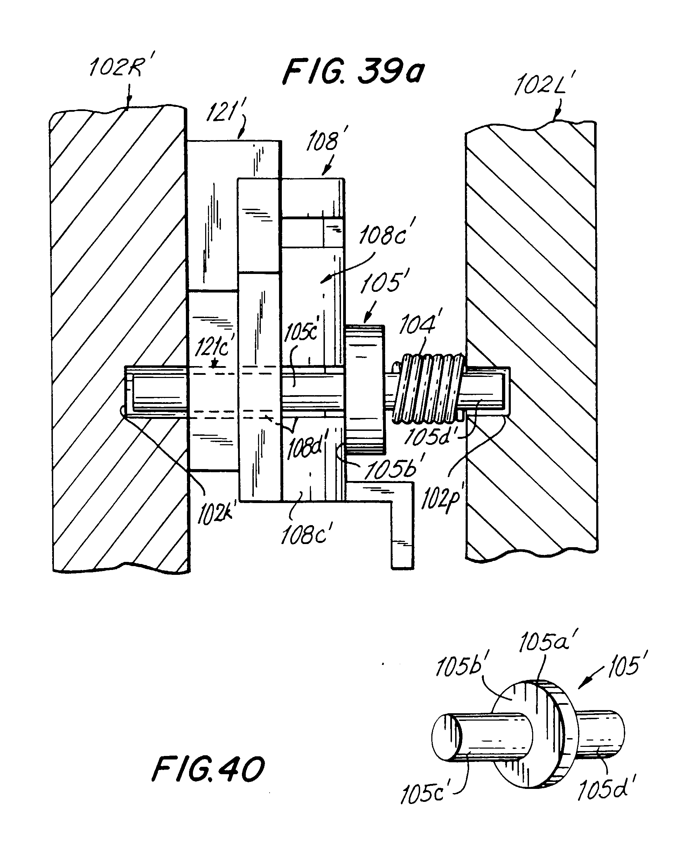

Referring additionally now to Figs. 39a, 39b and 40, channel lock pin 105' provides means for locking the rear channel 121' and comprises a cylindrical portion 105a' with camming surface 105b', and an axial shaft portion having ends 105c' and 105d'. When in the non-locking position channel lock pin 105' is located as illustrated in Fig. 39a. End 105d' of the shaft is disposed axially through spring 104' and into hole 102p' in the left frame portion 102L'. End 105c' is disposed through slot 108d' of the rear pusher bar 108'. When channel 105' is in the locking position as shown in Fig. 39b and 105c' is disposed through aperture 121c' in the rear channel 121', and hole 102k' in the right frame portion 102R'.

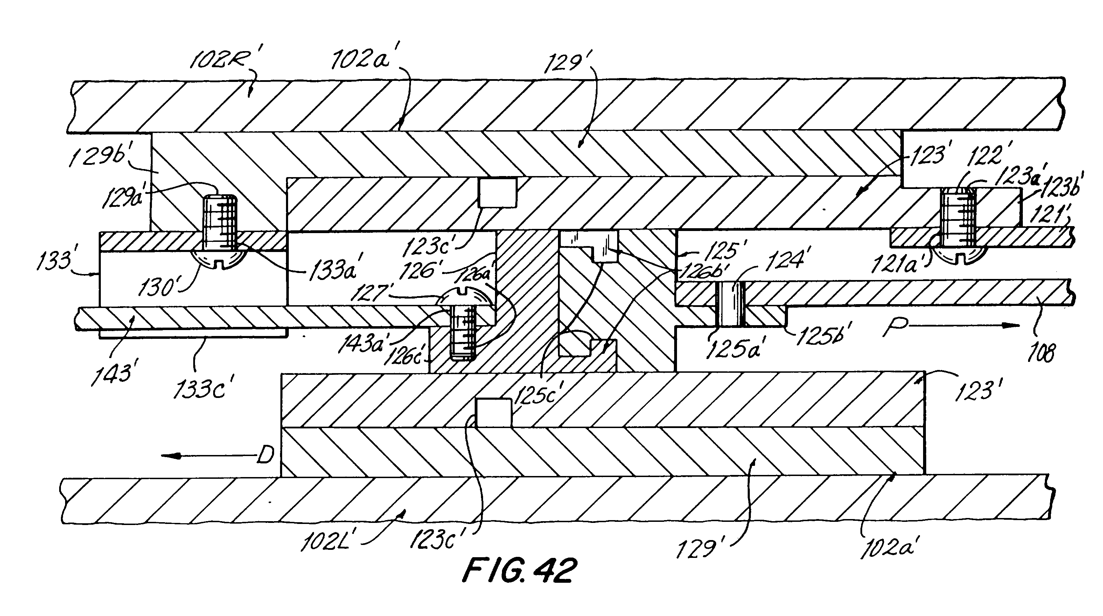

Fig. 41 illustrates the first transmission means (for transmitting linear movement to the clip advancing means) comprising the rear pusher bar 108', rear pusher bar tube 125', and front pusher bar tube 126', and the second transmission means comprising the rear channel 121', rear channel tube 123', and front channel tube 129'. The rear channel 121' is connected to rear channel tube 123' by means of screw 122' disposed through aperture 121a'. As can be seen additionally from Fig. 42, rear channel tube 123' has a proximal projection 123b' having an aperture 123a' for receiving screw 122'. Rear channel tube 123' provides means for transferring linear movement from the rear channel 121' to the front channel tube 129' and is generally cylindrical in shape having a hollow bore and an external circumferential notch 123c' for enabling the front channel tube to rotate.

Rear channel tube 123' is slidably mounted within the bore of front channel tube 129' which provides means for transferring linear motion from rear channel tube 123' to the front channel 133'. Front channel tube 129' has a distal projection 129b' having an aperture 129a' for receiving screw 130' which is the mounting means for the front channel 133'. Front channel tube is slidably mounted within the distal cylindrical chamber 102a' of frame 102'. Aperture 129a' in the front channel tube 129' receives pin 128' which projects into groove 123c' in the rear channel tube 123'.

Rear pusher bar 108' is connected to the proximal projection 125b' of the rear pusher bar tube 125' by means of pin 124' disposed through apertures 108a' in the rear pusher bar 108' and 125a' in the rear pusher bar tube 125'. Rear pusher bar tube 125' provides means for transferring linear movement from the rear pusher bar 108' to the front pusher bar tube 126' while permitting the front pusher bar tube 126' to rotate. Rear pusher bar tube 125' is generally of cylindrical shape and has an external circumferential notch 125c' for engaging proximal thrust collar 126b' in the front pusher bar tube 126'. Front pusher bar tube 126' has a distal projection 126c' having an aperture 126a' for receiving screw 127'. Screw 127' is for mounting the proximal end of front pusher bar 143' and is disposed through aperture 143a' in the front pusher bar.

Front pusher bar tube 126' transfers linear movement to pusher bar 143' from rear pusher bar tube 125'. Cooperating thrust collar 126b' and cylindrical notch 125c' provide means for allowing rotation of front pusher bar tube 126' relative to rear pusher bar tube 125'.

Rear pusher bar 108', rear pusher bar tube 125', front pusher bar tube 126', rear channel 121', front channel tube 129', and rear channel tube 123' are all slidable in the longitudinal direction. When the instrument 100' is actuated rear pusher bar 108', pusher bar tube 125' and front pusher bar tube 126' move proximally as indicated by arrow "P", and the rear channel 121', rear channel tube 123', and front channel tube 129' move distally as indicated by arrow "D". In addition to longitudinal movement, front pusher bar tube 126' and front channel tube 129' are rotatable around the instrument axis.

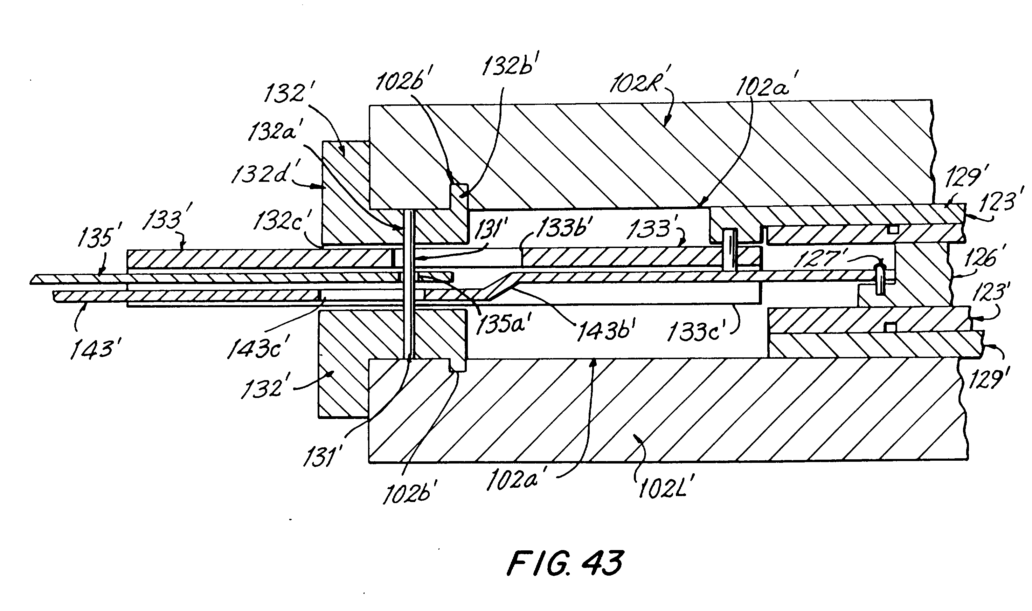

Referring additionally now to Fig. 43, collar 132' is generally cylindrical in shape having a radial aperture 132a', for receiving pin 131'; a circumferential detent 132b' for mounting into circumferential groove 102b' in the frame; a rectangular slot 132c' for receiving front channel 133', jaw blade 135', and front pusher bar 143'; and a distal portion 132d' which is located exterior to the frame. Collar 132' is rotatable but does not move longitudinally. The proximal end of front channel 133' is mounted to the front channel tube 129' by means of screw 130' disposed through apertures 133a' and 129a'. Front channel 133' has a slot 133b' to allow longitudinal movement without interference from pin 131'. When the instrument 100' is actuated, by pressing handle 112', front channel 133' moves distally. Slot 133b must therefore extend longitudinal for a distance sufficient to permit full distal movement of the front channel 133'.

Front channel 133', which provides means for closing jaws 135b', also has upper and lower guide rails 133c' which project transversely from the top and bottom of front channel 133', and which extend longitudinally. Guide rails 133c' serve as means to retain and align front pusher bar 143' and jaw blade 135' as well as means to close jaws 135b'.

Front pusher bar 143' is connected to the front pusher bar tube 126' by means of screw 127' disposed through apertures 143a' and 126a'. Front pusher bar 143' has a bend 143b' to widen the distance between it and the front channel 133' so as to accommodate jaw blade 135' disposed therebetween. Front pusher bar 143' has a slot 143c' to allow longitudinal movement without interference from pin 131'. When the instrument is actuated by pressing handle 112', front pusher bar 133' moves proximally. Slot 143c' must therefore extend longitudinally for a distance sufficient to permit full proximal movement of the front pusher bar 143'.

Jaw blade 135' has an aperture 135a' for receiving pin 131' and has a proximal end disposed within cylindrical collar 132'. Jaw blade 135' provides clip closing means.

As can be seen from Figs. 41, 42 and 43, when collar 132' is rotated for example by manually turning the distal portion 132d', jaw blade 135', front channel 133', and front pusher bar 143' are likewise rotated, as well as front tube channel 129' and front pusher bar tube 126'. Unlike front pusher bar 143' and front channel 133', jaw blade 135' does not also move longitudinally.

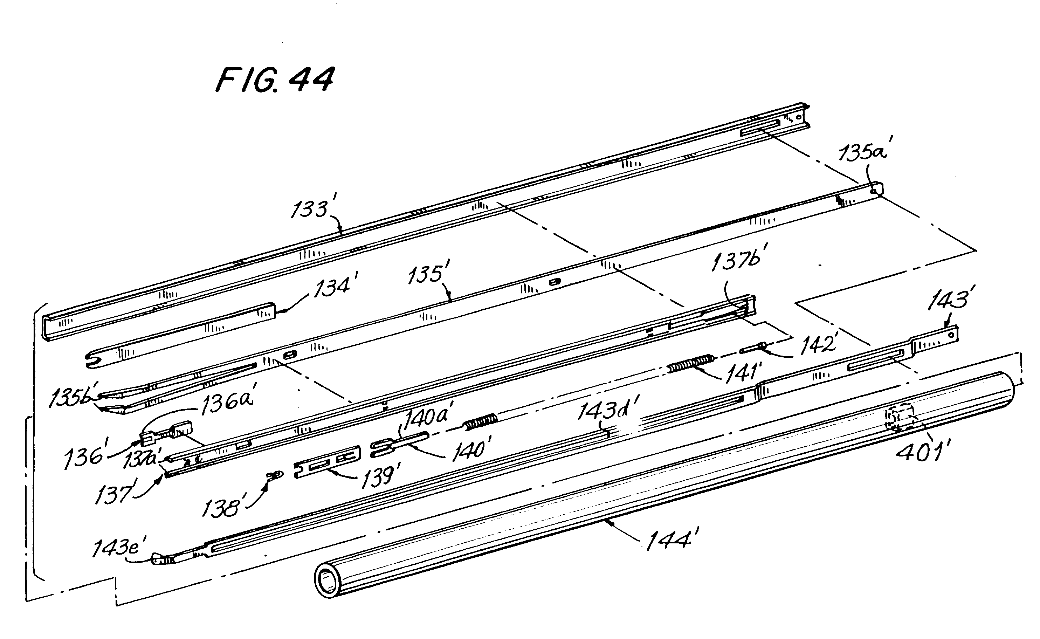

Referring now to Fig. 44, the endoscopic portion of the instrument comprises a cover tube 144' enclosing front pusher bar 143', spring anchor shaft 142', spring 141', rear pusher clip 140', front pusher clip 139', (optional) clips 138', clip carrier 137', safety stop 136', jaw blade 135', tissue stop 134', front channel 133', and sealing block 401'.

Cover 144' is an elongated tube fixed at its proximal end to collar 132'. Front pusher bar 143' is an elongated piece longitudinally disposed within cover 144'. In addition to features discussed above, front pusher bar 143' comprises an elongated longitudinal slot 143d', and inclined pusher tip 143e'. Carrier 137' is longitudinally positioned along the side of pusher 143' and provides a means for carrying surgical clips 138' which are disposed within the longitudinal guide rail 137a'. At its proximal end carrier 137' has a mounting post 137b' for spring anchor shaft 142'. The proximal end of spring 141' is mounted on anchor shaft 142', and the distal end of spring 141' is mounted to the proximal end of rear pusher clip 140'. Rear pusher clip 140' has a bar 140a' which rides in slot 143d' of the front pusher bar. The distal end of the rear pusher clip 140' contacts the proximal end of the front pusher clip 139', which engages and pushes the clips 138' distally.

Safety stop 136' is an escapement with prong members 136a' which project into the path of the clips 138' to limit the distal loading of the clips to one clip at a time. Safety stop 136' is attached to the distal end of carrier 137'.

Jaw blade 135' is disposed within the front channel 133' and has a pronged distal end with jaws 135b' which are flexibly movable toward each other. When the instrument is actuated the jaws are forced together by the camming action of the guide rails 133c' of the distally moving front channel 133'. Tissue stop 134', which is fixed to the side of the jaw blade 135' prevents the blood vessel or other tissue from proximally moving beyond the jaws 135b'.

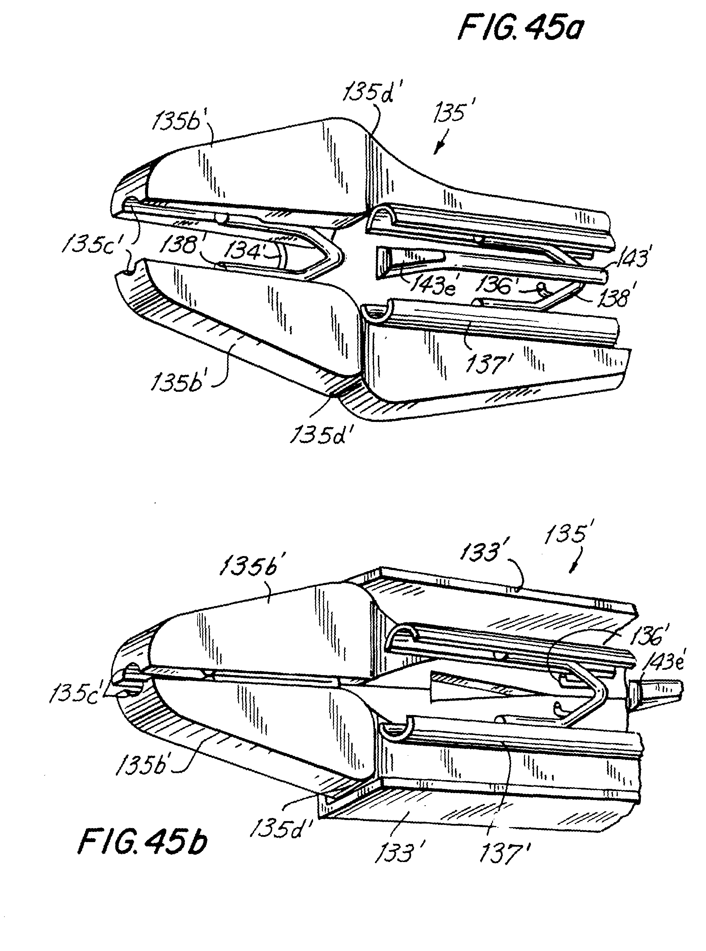

Figs. 45a and 45b illustrate a clip located between jaws 135b'. Grooves 135c' facilitate the proper alignment and positioning of the clip. Jaws 135b' also have camming surfaces 135d' which are contacted by the distally moving guide rails 133c' thereby forcing the jaws (and the clip 138') into closed position. The jaws blade 135' does not move longitudinally.

When handle 112' is pressed, it pivots clockwise thereby pivoting levers 110' and 113', and link 109' (which pivots counterclockwise) and link 114' (which pivots clockwise). Link 109' pushes the rear pusher bar 108' longitudinally in the proximal direction, and link 114' pushes the rear channel 121' longitudinally in the distal direction. Rear pusher bar 108' pulls the front pusher bar in the proximal direction via rear and front pusher bar tubes 125' and 126' respectively. The front and rear pusher bar tubes 126' and 125' are coupled so as to permit rotation of the front pusher bar tube 126' relative to the rear pusher bar tube 125'. The rear channel 121' pushes channel 133' distally via rear channel tube 123' and front channel tube 129'. The front and rear channel tubes 129' and 123' are coupled so as to permit rotation of the front channel tube 129' relative to the rear channel tube 123'. When channel 133' advances distally, it cams the jaws of jaw blade 135' into the closed position, thereby closing a surgical clip.

As the rear pusher bar 108' is being pushed proximally to the rear of the instrument, offset flap 108e' rides over the stop latch portion 116c' of spring 116', and when the offset flap passes proximally beyond the latch 116c', said latch 116c' then springs upward and engages edge 108g' of the rear pusher bar 108'. The pusher bar is then locked and prevented from going distally forward. Furthermore, when the rear pusher bar is moved to a proximal position, channel lock pin is permitted to extend further towards 102R'. When the handle 112' is subsequently released, the rear channel 121' is allowed to return proximally to its original prefiring position. When it does so, aperture 121c' becomes aligned with aperture 102k' in the right frame 102R', and the lock pin slides therethrough, locking the actuating mechanism so that the handle cannot be pressed again. The reason for this safety locking mechanism is that, having already closed a clip 138', jaws 135b' are not yet chambered with another clip. As a precautionary measure, lock pin 105' prevents the operating surgeon from mistakenly closing the jaws when they are empty.

To release the lock mechanism, the surgeon pushes the exposed pushing surface 115f' of the slide member 115' forward in the distal direction. Cam member 115a' then presses down the camming surface 116b' of spring 116'. This pushes down latch 116' and disengages the rear pusher bar 108', which slides forward. When rear pusher bar 108' slides forward the inclined camming surface 108c' wedges the lock pin 105' out of engagement with apertures 102k' and 121c', thereby unlocking the actuation mechanism. Furthermore, as rear pusher bar 108' springs forward the front pusher bar 143' also moves forward and pushes another clip 138' into position between jaws 135b'. The instrument 100' may then be reactuated to apply another clip.

A feature of this invention is that the transmission system transmits linear, longitudinal motion to the endoscopic portion of the instrument, while allowing the endoscopic portion of the instrument to rotate relative to the frame and actuating mechanism. This is accomplished by means of the couplings between front and rear pusher bar tubes 126' and 125', between front channel tube and rear channel tube 129' and 123', and between the collar 132' and the frame 102'.

In the above described embodiment the endoscopic portion is not detachable. However, a further alternative embodiment of the present invention comprises a disposable and replaceable endoscopic portion which is detachable from a reusable frame.

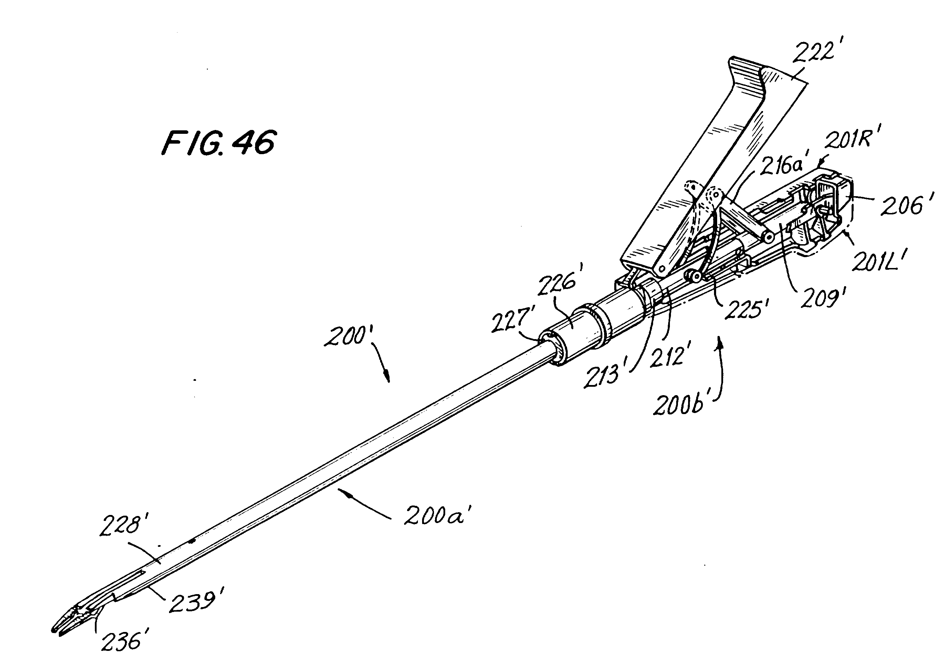

Fig. 46 shows a cutaway perspective view of the alternative embodiment of the present invention 200' which generally comprises a reusable actuating body 200b' supporting replaceable and disposable endoscopic portion 200a'. Included are means for actuating the instrument, transmission means, means for applying a surgical clip to a blood vessel or the like, means for locking the instrument, and means for repositioning another clip and unlocking the mechanism.

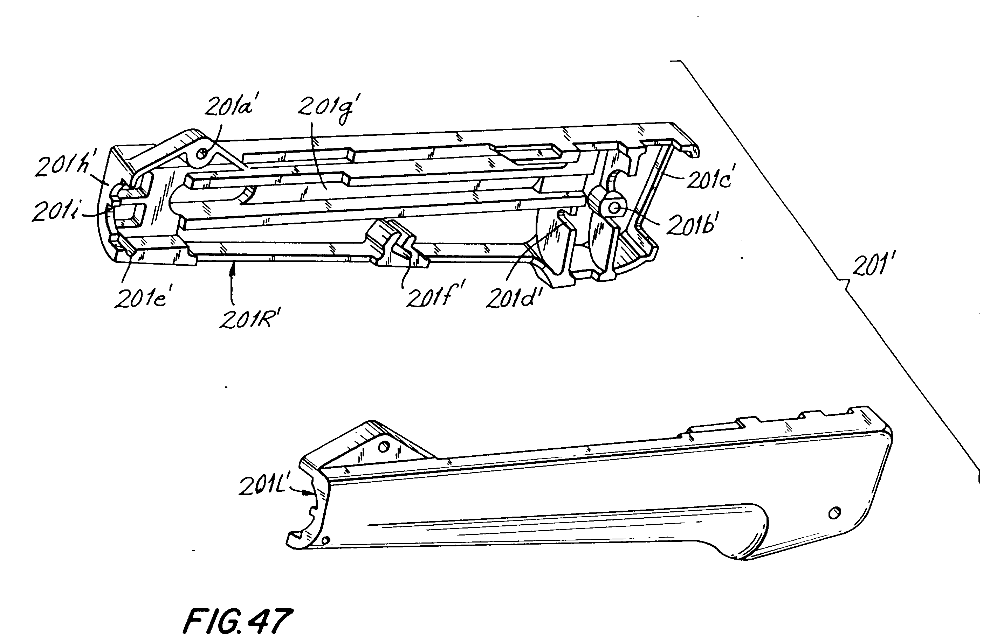

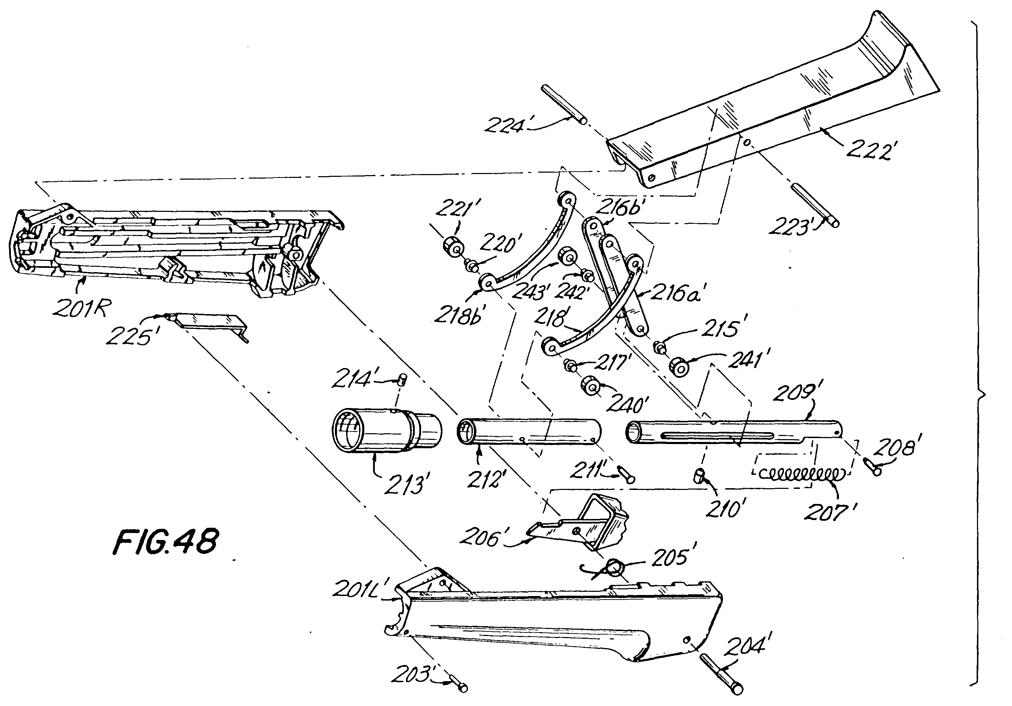

More particularly, referring to Figs. 46, 47 and 48, frame 201' comprises a left frame portion 201L' and right frame portion 201R'. These portions may be either cast or machined pieces of polymeric resin or metal. The left and right portions may optionally be fastened together by means of fastening screws although rivets, welds, adhesives, or other means of joining the frame portions may be used. Frame 201' is elongated and is of overall size and shape convenient for being held in the hand. Frame 201' has an interior surface defining a distal opening 201h', a proximal opening 201c', an interior transmission guideway 201g', apertures 201a' and 201b' for receiving pins 224' and 204' respectively, backstop notch 201d' for spring 205', bottom notch 201e', mounting notch for spring 225', and distal opening detents 201i'.

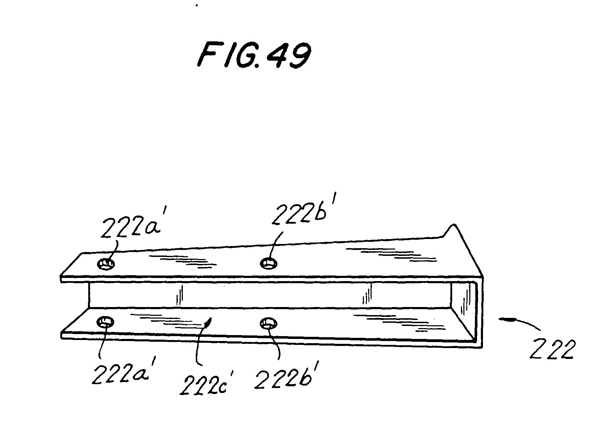

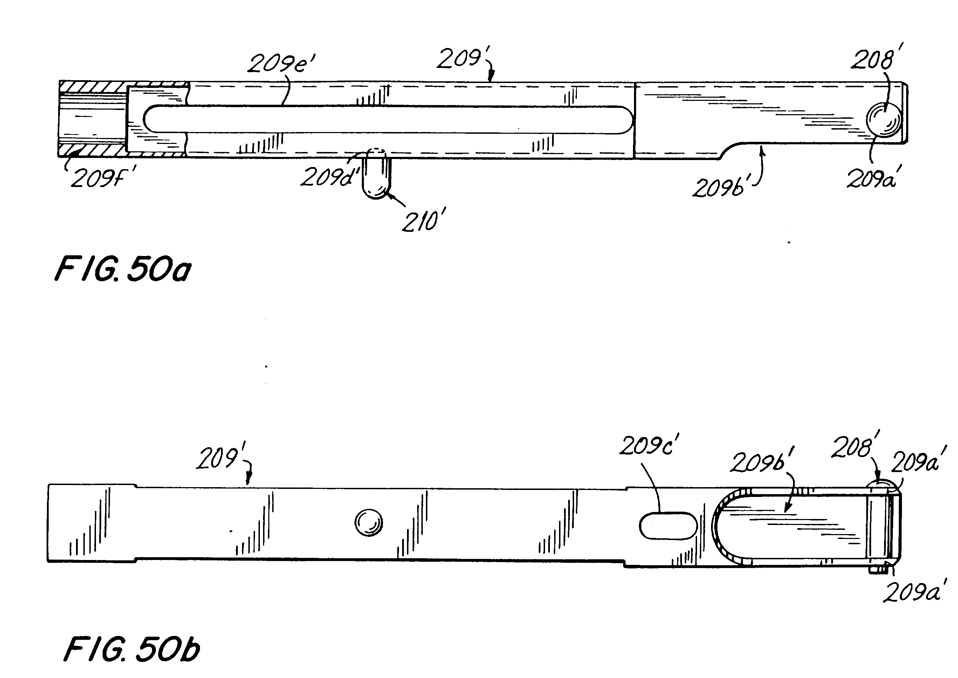

Referring additionally to Figs. 49, 50a, 50b, 51, 51a, 52 and 53, handle 222' is an elongated piece which is pivotably mounted to the distal end of frame 201' by means of pin 224' which is disposed through aperture 222a' in the handle and 201a' in the frame. Aperture 222b 'is adapted to receive link pivot pin 223'. Elongated cavity 222c' is adapted to receive pusher links 216' and 216a', and channel links 218' and 219'.

Channel links 218a' and 218b' preferably are curved elongated pieces having upper apertures for receiving pin 223', and lower apertures for receiving pins 217' and 220' respectively. Channel links 218a' and 218b' provide means for transferring movement from the handle 222' to the channel tube 212', and act as a load limiter as well.

Pusher links 216a' and 216b' are elongated pieces having apertures for receiving pin 223' and apertures for receiving pins 215' and 242' respectively. Pusher links 216a' and 216b' provide means for transferring movement from the handle 222' to the pusher tube 209'.

Pins 215', 217', 220' and 242' each comprise a disc like center portion, and two shafts projecting axially therefrom. The inwardly projecting shaft is adapted to be received into the respective aperture of the channel or pusher link, and the oppositely projecting shaft is adapted to be received into the axial bore of the respective roller bearings 241', 240', 221', and 243'. The inwardly projecting shafts of pins 217' and 220' are received into apertures in the channel tube 212, whereas the inwardly projecting shafts of pins 215' and 242' are disposed through the side slots of the pusher tube 209'.

The roller bearings 240', 221', 241', 243' are adapted to slide along the guideways 201g' in frame 201'.

Pusher tube 209' provides a first transmission means for transmitting linear movement to the pusher bar 229' and comprises an elongated tubular piece located within the transmission guideway 201g'. At its proximal end pusher tube 209' has apertures 209a' for receiving cross pin 208', and proximal terminal notch 209b'. Pusher tube 209' also comprises a bottom slot 209c' for engaging release button 206', an aperture 209d' for receiving pusher tube camming pin 210', side slots 209e' for receiving pins 215' and 242', and an interior circumferential projection 209f' at the distal end of the pusher tube for engaging the proximal end of the pusher 229' (see below).

Channel tube 212' provides a second transmission means for transmitting linear movement to channel 238' and comprises a proximal aperture 212a' for receiving pin 211', apertures 212b' for receiving pins 217' and 220', bottom aperture 212c' for engaging spring clip 225', and proximal slot 212d' for receiving camming pin 210'. Pusher tube 209' is slidably mounted within channel tube 212' and the two are biased into alignment by means of spring 207' which is axially disposed within pusher tube 209' and which is attached at its proximal end to pin 208' and at its distal end to pin 211'. Channel tube 212' further has an interior circumferential projection 212e' at the distal end of for engaging the proximal end of channel 233'.

Leaf spring or spring clip 225' is a catch means for locking the channel tube in the distal position after the instrument has been actuated. Spring clip 225' has a distal end with angled portion 225a' for being mounted in notch 201f' of the frame. At its distal end, resilient spring clip 225' has a catch 225b' which is engagable with aperture 212c' of the channel tube 212'. Spring clip 225' has a camming surface 225c' which is contacted by pusher tube camming pin 210' when the pusher tube moves in the distal direction, thereby depressing the spring clip 225' and disengaging and unlocking the channel tube 212'.

Release button 206' is pivotally mounted at the proximal end of frame 201' by means of pivot pin 204' which is disposed through aperture 206a' and 201b'. Release button 206' has a proximal end 206b' which projects through aperture 201c' in the frame, and a catch 206c' at its distal end for engaging aperture 209c' of the pusher tube 209'.

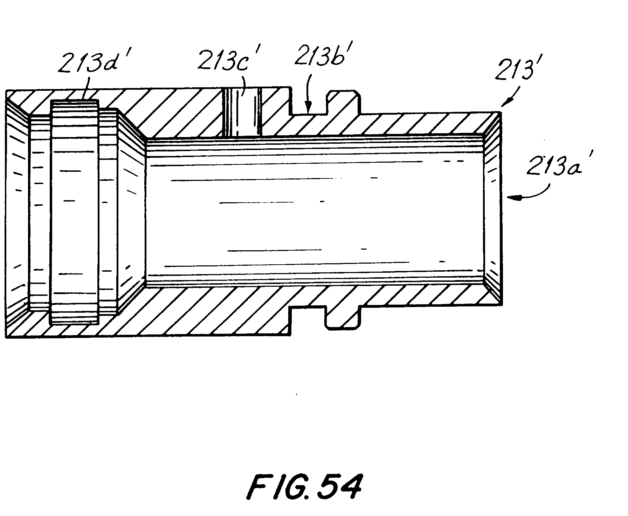

Referring additionally now to Fig. 54, outer tube 213' is adapted to be received into distal access opening 201h' of the frame. Outer tube 213' has a proximal opening 213a', a circumferential outer notch 213b' which is adapted to receive detent 201i' in the distal opening of the frame, an aperture 213c' for receiving pin 214', and a circumferential inner notch 213d' at the distal end of the outer tube for engaging and interlocking with the collet 227'.

Pin 214' is received into aperture 213c' of the outer tube and projects a small distance beyond the outer surface thereof. It provides a contact surface to engage the interior notches of the sleeve 226' as discussed below.

The above described portion of the alternative embodiment is the reusable portion 200b'. The replaceable endoscopic portion 200a' described below is adapted to be received into the distal end of the reusable portion 200b' and to project outwardly therefrom. The endoscopic portion 200a' is rotatable relative to the reusable portion 200b'.

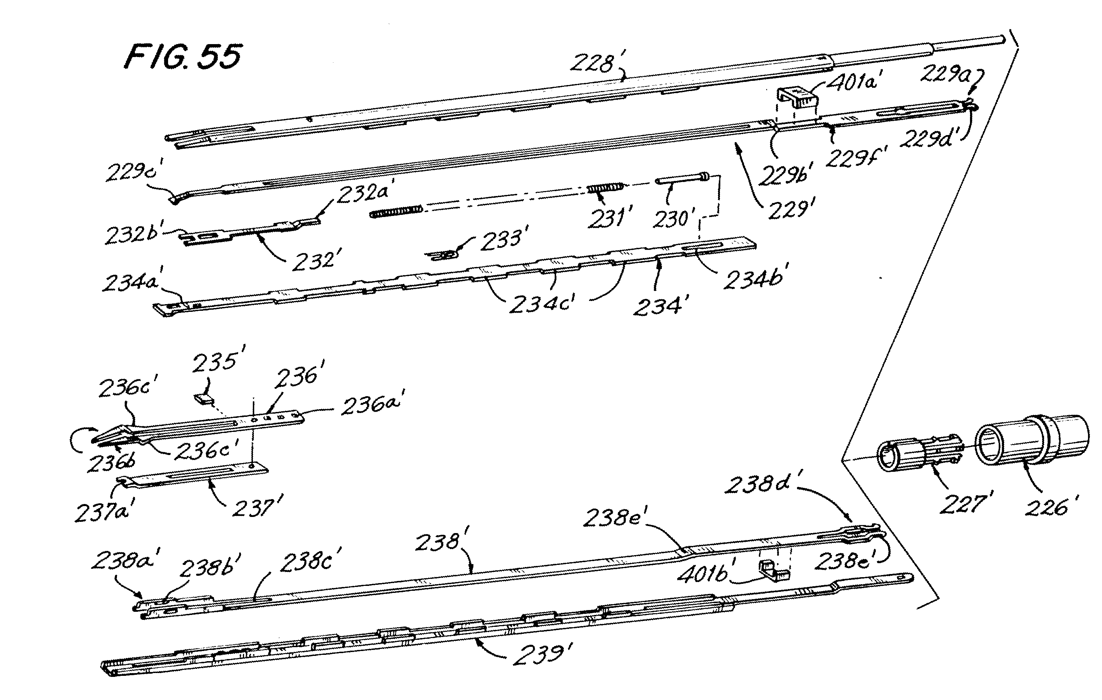

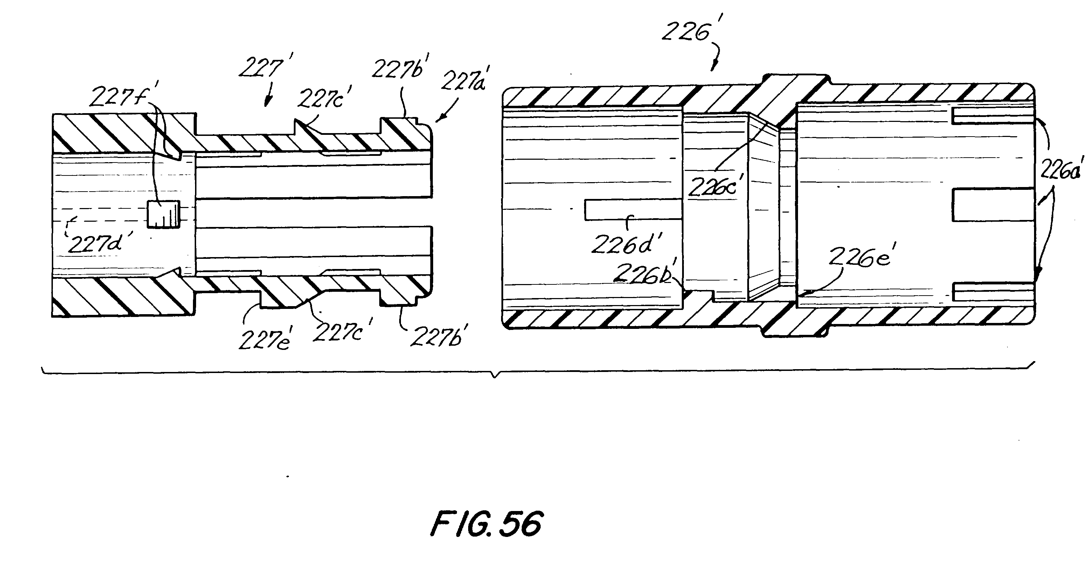

Referring additionally now to Figs. 55 and 56, the replaceable portion 200a' includes a coupling which comprises sleeve 226' and collet 227'. The coupling is adapted to connect the endoscopic portion to the non-endoscopic instrument body portion while permitting rotation of the endoscopic portion. Sleeve 226' is a tubular piece having several longitudinal notches 226a' on the interior surface of the proximal end for engaging the protruding portion of pin 214'. The pin 214' and notches 226a' cooperate so as to form click-stop settings wherein the sleeve can rotate to any of several positions and be temporarily seated in the chosen position. Sleeve 226' has slot 226e' and detent 226b' which are adapted to cooperate with the collet 227' to retain the collet 227' within the sleeve 226' once the collet 227' has been inserted. Detent 226b' serves as a backstop by abutting the distal surface of projection 227e' in the collet. Key 226d' on the inside surface of sleeve 226' is adapted to fit into longitudinal spline 227d' in the outside surface of collet 227'. Camming surface 226c' is adapted to push against surface 227c' in the outer surface of the collet 227'.

The proximal portion of collet 227' comprises several proximally extending prongs 227a', each prong having on its outer surface a projection 227b' for engaging and locking into circumferential notch 213d' on the interior surface of the outer tube 213'. Once inserted into sleeve 226', collet 227' locks into place by means of projecting surface 227e' which abuts detent 226b' in the sleeve. Projections 227f' lock into cooperating notches in the cartridge halves 228', 229'. When camming surfaces 227c' on the exterior surface of the prongs 227b' are contacted by camming surface 227c' in the sleeve as for example when the sleeve 226' and collet 227' are pushed together, the prongs 227b' are biased closer to the axial center of the collet 227', thereby enabling them to disengage from the outer tube.

The endoscopic portion is axially disposed through the collet and sleeve, and connects to the actuating and transmission mechanisms described above. As can be seen from Fig. 55, the endoscopic portion comprises upper and lower cartridge portions 228' and 239', and disposed within the cartridge portions are pusher bar 229', clip pusher 232', clip feed spring 231', spring anchor shaft 230', bottom clip track 234', capture plate 235', jaw blade 236', tissue stop 237', channel 238', clip 233' and sealing block 401'.

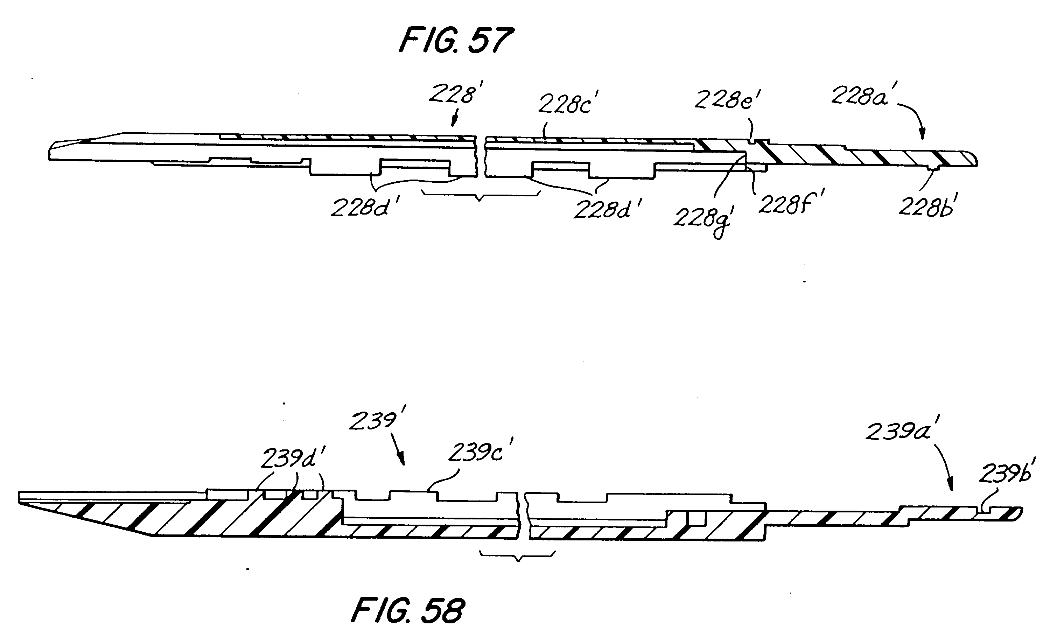

Referring additionally now to Figs. 57 and 58, upper cartridge portion 228' comprises an elongated relatively small diameter piece which, in addition to lower cartridge portion 239', houses the endoscopic portion of the transmission and clip applying mechanisms. The cartridge may be constructed of polymeric material suitable for surgical procedures and has a width of about 0.3 inches for practical use in endoscopic or laparoscopic surgical procedures. Upper cartridge portion 228' comprises a proximal end 228a' having a projection 228b' for engaging corresponding notch 239b' in the lower cartridge portion, notches 228e' for engaging corresponding projections 227f' in the collet, stopping surface 228f' for limiting the distal or forward movement of the pusher bar 229' by engaging projections 229f', stopping surface 228g' for limiting the rearward or proximal movement of the pusher bar 229' by engaging bend 229b', rectangular projections 228d' for alternating alignment with projections 239c' in the lower cartridge portion 239', and guide way 228c' for aligning the pusher bar 229'.

Lower cartridge portion 239' comprises a proximal portion 239a' having a notch 239b' for receiving projection 228b', projections 239c', and projections 239d' for engaging the corresponding apertures in the jaw blade 236'.

Channel 238' is an elongated piece having a distal end with camming portions 238a' for contacting the jaws 236b' and pushing them into a closed position. Slots 238b' are for receiving capture plate 235'. Channel 238' has an aperture 238c' for receiving projections 239d of the bottom portion of the cartridge 239', bend 238e', and proximal prongs 238d' having notches 238e' for engaging projection 212e' in the channel tube. Prongs 238' are resiliently flexible towards each other. Tissue stop 237' has a proximal end 237a' to protect the blood vessel or other tissue from entering too far between the jaws 236b'.

Jaw blade 236' has a proximal end with apertures 236a' for engaging projections 239d' in the cartridge thereby aligning and firmly seating the jaw blade 236'. Jaws 236b' at the distal end have camming surfaces 236c' which are contacted by the camming surfaces 238a' of the channel. When the channel moves in the distal direction the jaw blades 236b' are cammed into the closed position, thereby closing a clip positioned within said jaws.

Bottom track 234' for clips 233' is an elongated flat piece having a distal end with projections 234a' which serve as an escapement means to limit the feeding of clips 233' to one clip at a time. Bottom track 234' has a mounting post 234b' for the spring anchor 230', and flat side projections 234a' for fitting and alignment in the cartridge 228'.

Clip pusher 232' has a distal end with prongs 232b' adapted to fit around any distally push clips 233' forward. Clip pusher 232a' has a proximal post 232a' for mounting spring 231'.

Spring 231' is axially mounted at its proximal end to anchor 230', and at its distal end to clip pusher 232'. Spring 231' resiliently moves the clip pusher forward in the distal direction.

Pusher bar 229c' provides a means for advancing the clips into the jaws. Distal end 229c' pushes the clips forward one at a time from track 234' to a position between the jaws.

Pusher bar 229' has a bend 229b' which abuts stopping surface 228g' to limit the proximal movement of he pusher bar, projections 229f' which abut stopping surface 228f' for limiting distal movement of the pusher bar 229', and a proximal end with resilient prongs 229a', each prong having a notch 229d' for engaging projection 209f' in the pusher tube. The resiliency of the prongs allow them to bend inward sufficiently to enable them to snap into place when the endoscopic portion of the instrument is inserted into the reusable body.

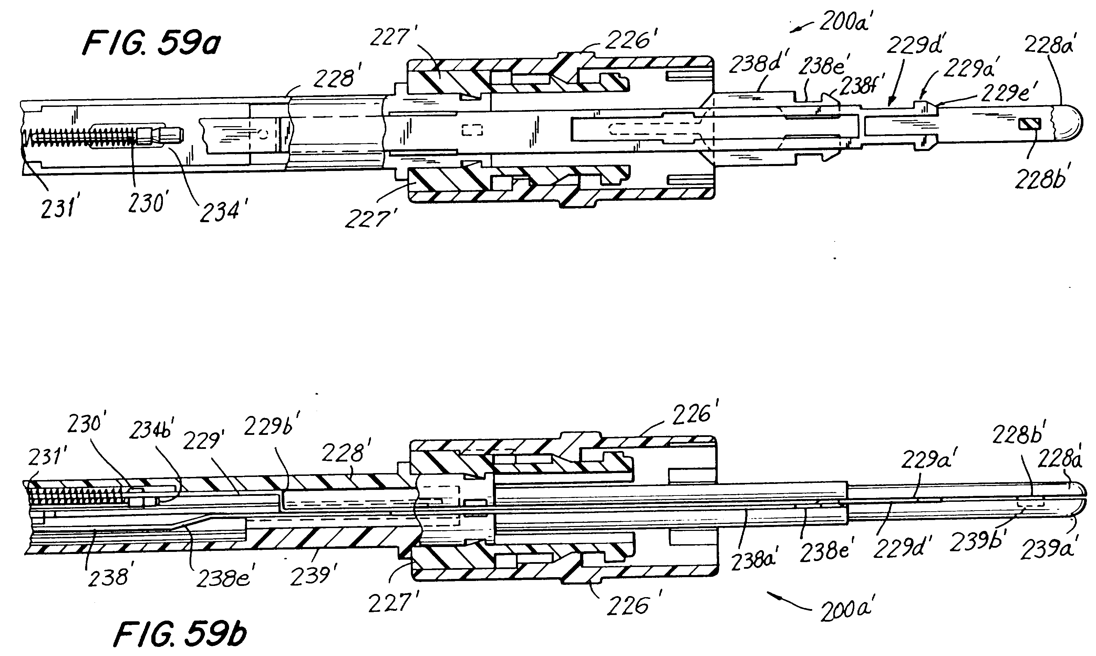

Referring additionally now to Fig. 59a, which shows a top sectional view of the proximal coupling end of the endoscopic portion 200a', and to Fig. 59b which shows the same portion in side view, the cartridge portions 228' and 239' which house the endoscopic portion of the transmission means, are disposed axially through the collet 227' and sleeve 226'. As can be seen from the illustration pusher bar 229' and channel 238' are sandwiched between the cartridge portions, but have proximal ends 229a' and 238d' which, being slightly wider than the cartridge section 239a' and 228a', protrude slightly from the sides. Inclined proximal edges of the pusher bar (229e') and the channel (238f') allow the respective prongs to be biased inward while the endoscopic portion 200a' is being inserted into the reusable body 200b'. The proximal portions 229a' and 238a' will then snap into place as channel tube projection 212e' engages notches 238e' and pusher tube projection 209f' engages notches 229d', the prongs 229a' and 238d' thereupon resiliently expanding to their original position. The proximal movement of pusher tube 209' will draw the pusher bar 229' rearward in the proximal direction, and the distal movement of channel tube 212' will push the channel 238' forward.

Initially, the clip applier of the present invention is in the locked position, i.e., there is no clip loaded between the jaws. The jaws are biased open and are free to cam between the opera and closed positions. This facilitates insertion of the endoscopic portion into an endoscopic tube or into the body since the jaws can cam partially closed, thereby avoiding interference with the positioning of the instrument.

When the clip applier has been properly positioned, the user may release the first and second transmission means from the locked position by pressing the release button (206'). This disengages the first transmission means (pusher bar 209') which slides forward. The first transmission means comprises means to release the catch from the second transmission means. For example, in the first described embodiment the rear pusher bar has an inclined camming surface 108c' to push aside channel lock pin 105' which locks the second transmission means. In the alternative embodiment pusher tube 209' (the first transmission means) has a depending pin 210' which, upon being carried forward, depresses leaf spring 225', thereby releasing the catch 225b' from the channel tube 212' (second transmission means).

The first transmission means of both embodiments transfers motion to the pusher bar (143' and 229') which has a distal pusher end located behind the distal most clip in the array. When the pusher bar (i.e., the clip advancing means) moves forward the distal most clip is advanced to the jaws.

The second transmission means, upon being released, returns backward, thereby pulling the channel (133' in the first described embodiment; 238' in the alternative embodiment). The instrument is now ready to be actuated for clip application.

The endoscopic portion of the instrument 200a' may be rotated relative to the reusable body 200b' by manually turning sleeve 226'.

As in the previously described first embodiment, the clip applier of the alternative embodiment is actuated by pressing the handle. When handle 201' is pressed pusher links 216a' and 216b' move pusher tube 209' proximally to the rear of the instrument thereby repositioning the pusher bar behind another clip, and channel links 218a' and 218b' move the channel tube 212' forward thereby causing the jaws to be closed for applying the surgical clip. The distally moving channel tube 212' pushes channel 233' forward, thereby camming the jaw blade 236' into a closed position for applying the surgical clip. The pusher tube 209' is locked in the proximal position when catch 206c' of the release button 206' engages aperture 209c' in the pusher tube. The channel tube 212' is locked in the distal position when catch 226b' of the leaf spring engages aperture 212c' of the channel tube.

The instrument remains locked until the release button 206 is pressed, thereby unlocking the instrument as explained above.

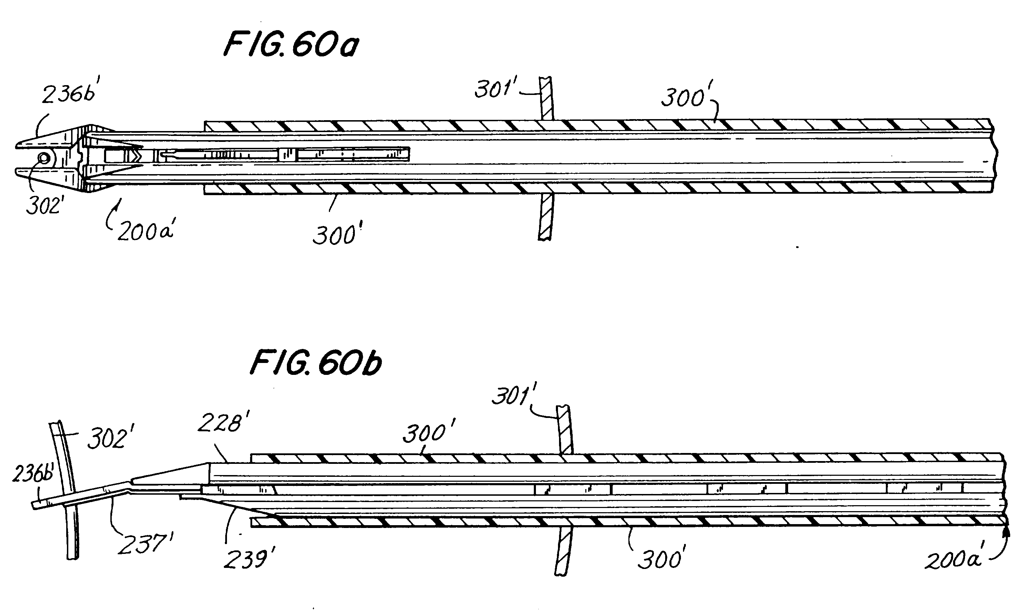

Figs. 60a and 60b illustrate the apparatus of the present invention in conjunction with the cannula 300', or endoscopic guide tube, of a trocar to apply a surgical clip to blood vessel 302'. (Trocars generally comprise a cutting tip, cannula, and valve means for sealing the cannula). The trocar is used to penetrate the skin 301' of a patient and is inserted into the patient's body. Upon withdrawal of the cutting tip (not shown) of the trocar, the endoscopic portion of the apparatus 200a' is inserted axially through the cannula 300' and maneuvered to the operating site where jaws 236b' engage blood vessel 302'. The endoscopic portion is in sealing engagement with the valve means to prevent entry or egress of gases.

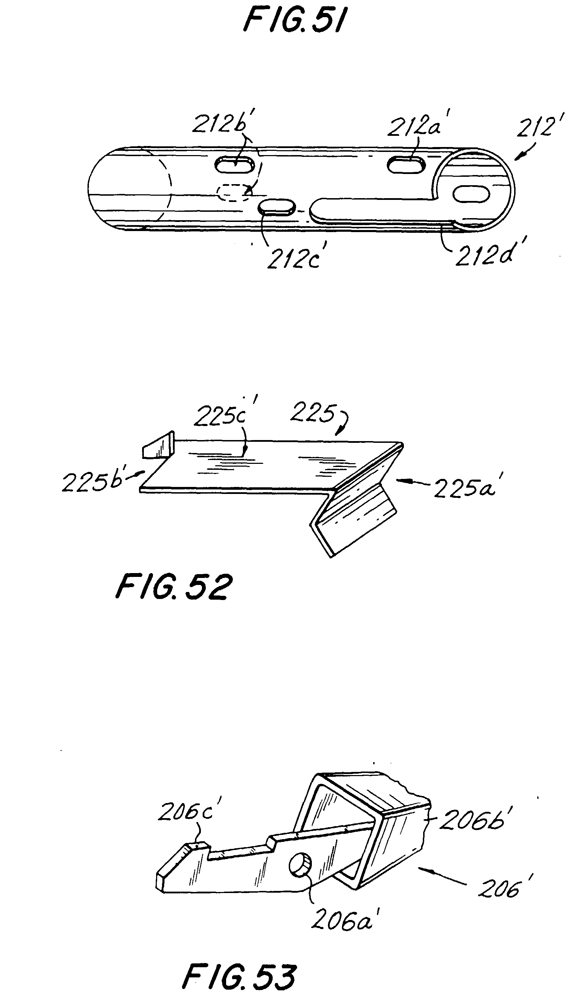

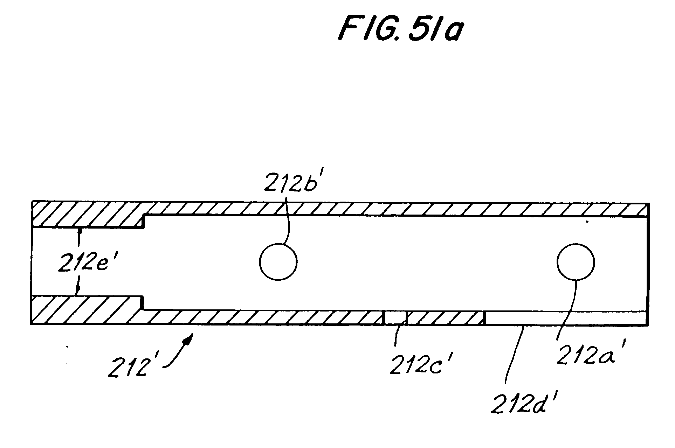

Optimally, the gaseous sealing means comprises sealing block 401', as illustrated in Fig. 51. Sealing block 401' comprises U-shaped upper and lower portions 401a' and 401b', respectively, which are positioned together so as to define an axially extending opening 401c' through which components such as channel 238' and pusher 229' may be disposed. The inner surface of opening 401c' is in close contiguity with the surface of the components disposed therethrough. A layer of silicone grease 402' may be employed to prevent gases from leaking between the surfaces. The sealing block 401' may be located within the cover tube 144' or the cartridge portions 228' and 239' of the embodiments disclosed herein. See, for example, Figs. 44 and 55.

While the above description contains many specifics, these specifics should not be construed as limitations on the scope of the invention, but merely as exemplifications of preferred embodiments thereof. Those skilled in the art will envision many other possible variations that are within the scope of the claims appended hereto.

## Claims

1. A surgical apparatus (10) comprising:
(a) a frame and at least one actuating handle movably mounted with respect to the frame;
(b) an elongate endoscopic section (14) having a proximal end connected to the frame;
(c) flexible opposing jaws (24) positioned at the distal end of the endoscopic section;
(d) a cam member (32, 133c) having a proximal end which extends into the frame and a distal end which at least partially surrounds the flexible opposing jaws; the apparatus being characterised by:
(e) means for storing a plurality of clips;
(f) the cam member being movably positioned within the elongated section between a proximal position and a distal position wherein the flexible opposing jaws are cammed (50, 52, 135d) into closure;
(g) a pusher member (78, 143) having a proximal end which extends into the frame and a distal end, the pusher member being movably positioned within the elongated section between a proximal position and a distal position whereby a clip is moved within the flexible opposing jaws;
(h) a spring (140, 207) positioned within the frame and abutting the cam member and the pusher member, the spring biasing the cam member proximally and the pusher member distally; and
(i) the endoscopic section is mounted to the frame in a way (132, 170) which permits manual manipulation at the frame to rotate the jaws around the axis of the endoscopic section, relative to the frame.

2. The apparatus of claim 1, wherein said actuating handle is operatively connected to said cam member and said pusher member within said frame such that movement of the actuating handle with respect to the frame moves the cam member and the pusher member against the bias of the spring.

3. The apparatus of claim 1 or 2, further comprising a first linkage (120, 122) mounted to the actuating handle and the pusher member, and a second linkage (116, 118) mounted to the actuating handle and the cam member.

4. The apparatus of claim 3, wherein the first linkage is configured to translate an arcuate movement of the actuating handle with respect to the frame into linear movement of the pusher member with respect to the frame, and the second linkage is configured to translate the arcuate movement of the actuating handle into linear movement of the cam member with respect to the frame.

5. The apparatus of any one of the preceding claims wherein the frame defines a stationary handgrip (100) and wherein the actuating handle (18) is movable between a first position spaced from the stationary handgrip and a second position in proximity to the stationary handgrip.

6. The apparatus of claim 5, wherein said actuating handle is operatives connected to said cam member and said pusher member within said frame such that movement of the actuating handle from the first position to the second position translates to movement of the cam member distally and the pusher member proximally relative to the frame.

7. The apparatus of claim 5 or 6, wherein the spring (140, 207) is a tension spring and movement of the actuating handle from the first position to the second position translates to stretching of the tension spring.

8. The apparatus of any one of the preceding claims wherein the pusher member includes a pusher tube (209) movably positioned within the frame and the cam member includes a cam tube (212) movably positioned within the frame, and the spring (207) acts on the pusher tube and the cam tube, biasing the pusher tube distally and the cam tube proximally.

9. The apparatus of any one of the preceding claims, wherein the endoscopic section defines a longitudinal axis and is rotatable with respect to the frame, and further wherein, regardless of the angular orientation of the elongated section relative to the frame, the spring retains a fixed angular orientation with respect to the frame.

10. The apparatus of any one of the preceding claims, further comprising a seal block (401) positioned within the elongated section and co-operating with the cam member and the pusher member to inhibit gas flow through the elongated section.

11. The apparatus of any one of the preceding claims, further comprising a clip track (137) mounted within the elongated section and a plurality of clips (138) positioned within the clip track.

12. The apparatus of any one of the preceding claims, further comprising (i) a latch (150) positioned within the frame, the latch movable between a delatched position and a latched position wherein the latch (158) engages the pusher member (134) and restrains the pusher member in a proximal position against the bias of the spring, and (ii) a trigger (16) mounted to the frame and movable from a first position to a second position to move the latch from the latched to the delatched position.

13. The apparatus of any one of the preceding claims, wherein the pusher member includes a pusher bar and a pusher tube, and wherein the cam member includes a cam channel and a cam tube.

14. The apparatus of any one of the preceding claims, further comprising a mechanism for detachably mounting the endoscopic section to the frame, a first part (227) of the mechanism being positioned at the proximal end of the elongated section and a second part (226) of the mechanism being positioned within the frame.

## Patentansprüche

1. Chirurgische Vorrichtung (10) umfassend:
(a) einen Rahmen und zumindest einen Betätigungsgriff, der bewegbar in bezug auf den Rahmen angebracht ist;
(b) einen langgestreckten endoskopischen Abschnitt (14) mit einem proximalen Ende, das mit dem Rahmen verbunden ist;
(c) biegsame, gegenüberliegende Klemmbacken (24), die am distalen Ende des endoskopischen Abschnittes positioniert sind;
(d) ein Nockenelement (32, 133c) mit einem proximalen Ende, das sich in den Rahmen erstreckt, und einem distalen Ende, das zumindest teilweise die biegsamen, gegenüberliegenden Klemmbacken umgibt;
wobei die Vorrichtung gekennzeichnet ist durch:
(e) eine Einrichtung, um eine Mehrzahl von Klammern aufzubewahren;
(f) die bewegbare Anordnung des Nockenelementes innerhalb des langgestreckten Abschnittes zwischen einer proximalen Position und einer distalen Position, wobei die biegsamen, gegenüberliegenden Klemmbacken nockenartig (50, 52, 135d) in eine Schließstellung bewegt werden;
(g) ein Schieberelement (78, 143) mit einem proximalen Ende, das sich in den Rahmen erstreckt, und einem distalen Ende, wobei das Schieberelement bewegbar innerhalb des langgestreckten Abschnittes zwischen einer proximalen Position und einer distalen Position angeordnet ist und eine Klemme innerhalb der biegsamen, gegenüberliegenden Klemmbacken bewegt wird;
(h) eine Feder (140, 207), die innerhalb des Rahmens angeordnet ist und gegen das Nockenelement und das Schieberelement anstößt, wobei die Feder das Nockenelement proximal und das Schieberelement distal vorspannt; und
(i) wobei der endoskopische Abschnitt an dem Rahmen derart (132, 170) angebracht ist, daß eine manuelle Betätigung am Rahmen ermöglicht ist, um die Klemmbacken um die Achse des endoskopischen Abschnittes relativ zum Rahmen zu drehen.

2. Vorrichtung gemäß Anspruch 1, wobei der Betätigungsgriff betriebsmäßig mit dem Nockenelement und dem Schieberelement innerhalb des Rahmens so verbunden ist, daß eine Bewegung des Betätigungsgriffes in bezug auf den Rahmen das Nockenelement und das Schieberelement gegen die Vorspannung der Feder bewegt.

3. Vorrichtung gemäß Anspruch 1 oder 2, weiter umfassend eine erste Verbindung (120, 122), die am Betätigungsgriff und dem Schieberelement angebracht ist, und eine zweite Verbindung (116, 118), die am Betätigungsgriff und dem Nockenelement angebracht ist.

4. Vorrichtung gemäß Anspruch 3, wobei die erste Verbindung gestaltet ist, um eine bogenförmige Bewegung des Betätigungsgriffes in bezug auf den Rahmen in eine lineare Bewegung des Schieberelementes in bezug auf den Rahmen zu übertragen, und die zweite Verbindung gestaltet ist, um die bogenförmige Bewegung des Betätigungsgriffes in eine lineare Bewegung des Nockenelementes in bezug auf den Rahmen zu übertragen.

5. Vorrichtung gemäß einem der vorhergehenden Ansprüche, wobei der Rahmen einen feststehenden Handgriff (100) definiert und wobei der Betätigungsgriff (18) zwischen einer ersten Position vom stationären Handgriff beabstandet und einer zweiten Position nahe dem feststehenden Griff bewegbar ist.

6. Vorrichtung gemäß Anspruch 5, wobei der Betätigungsgriff betriebsmäßig mit dem Nockenelement und dem Schieberelement innerhalb des Rahmens so verbunden ist, daß eine Bewegung des Betätigungsgriffes von der ersten Position in die zweite Position sich in eine Bewegung des Nockenelementes in distaler Richtung und des Schieberelementes in proximaler Richtung relativ zum Rahmen überträgt.

7. Vorrichtung gemäß Anspruch 5 oder 6, wobei die Feder (140, 207) eine Zugfeder ist und eine Bewegung des Betätigungsgriffes von der ersten Position in die zweite Position die Zugfeder dehnt.

8. Vorrichtung gemäß einem der vorhergehenden Ansprüche, wobei das Schieberelement eine Schieberröhre (209) aufweist, die bewegbar innerhalb des Rahmens angeordnet ist, und das Nockenelement eine Nockenröhre (212) aufweist, die bewegbar innerhalb des Rahmens angeordnet ist, und die Feder (207) auf die Schieberröhre und die Nockenröhre wirkt, wobei sie die Schieberröhre in distaler Richtung und die Nockenröhre in proximaler Richtung vorspannt.

9. Vorrichtung gemäß einem der vorhergehenden Ansprüche, wobei der endoskopische Abschnitt eine Längsachse definiert und in bezug auf den Rahmen drehbar ist, und weiterhin ungeachtet der Winkelausrichtung des langgestreckten Abschnittes relativ zum Rahmen die Feder eine feststehende Winkelausrichtung in bezug auf den Rahmen aufrechterhält.

10. Vorrichtung gemäß einem der vorhergehenden Ansprüche, weiter umfassend einen Abdichtblock (401), der innerhalb des langgestreckten Abschnittes angeordnet ist und mit dem Nockenelement und dem Schieberelement zusammenwirkt, um einen Gasfluß durch den langgestreckten Abschnitt zu behindern.

11. Vorrichtung gemäß einem der vorhergehenden Ansprüche, weiterhin umfassend eine Klemmenschiene (137), die innerhalb des langgestreckten Abschnittes befindlich ist, und eine Mehrzahl von Klemmen (138), die innerhalb der Klemmenschiene angeordnet sind.

12. Vorrichtung gemäß einem der vorhergehenden Ansprüche, weiterhin umfassend
(i) eine Sperrklinke (150), die innerhalb des Rahmens angeordnet ist, wobei die Sperrklinke bewegbar ist zwischen einer gelösten Position und einer verriegelten Position, wobei die Sperrklinke (158) mit dem Schieberelement (134) in Eingriff ist und das Schieberelement in einer proximalen Position gegen die Vorspannung der Feder hält, und
(ii) einen Auslöser (16), der an dem Rahmen angebracht ist und von einer ersten Position in eine zweite Position bewegbar ist, um die Sperrklinke von der verriegelten in die gelöste Position zu bewegen.

13. Vorrichtung gemäß einem der vorhergehenden Ansprüche, wobei das Schieberelement eine Schieberstange und eine Schieberröhre aufweist, und wobei das Nockenelement eine Nockenrinne und eine Nockenröhre aufweist.

14. Vorrichtung gemäß einem der vorhergehenden Ansprüche, weiterhin umfassend einen Mechanismus, um lösbar den endoskopischen Abschnitt am Rahmen anzubringen, wobei ein erstes Teil (227) des Mechanismus an dem proximalen Ende des langgestreckten Abschnittes angeordnet ist und ein zweites Teil (226) des Mechanismus innerhalb des Rahmens angeordnet ist.

## Revendications

1. Appareil chirurgical (10) comprenant :
(a) un châssis et au moins une poignée d'actionnement montée d'une manière mobile par rapport au châssis;
(b) une section endoscopique oblongue (14) ayant une extrémité proximale reliée au châssis;
(c) des mâchoires opposées flexibles (24) positionnées à l'extrémité distale de la section endoscopique;
(d) un élément de came (32, 133c) ayant une extrémité proximale qui s'étend dans le châssis et une extrémité distale qui entoure au moins partiellement les mâchoires opposées flexibles;
l'appareil étant caractérisé par:
(e) un moyen pour stocker une pluralité de pinces;
(f) l'élément de came étant positionné d'une manière mobile dans la section oblongue entre une position proximale et une position distale où les mâchoires flexibles opposées sont amenées par effet de came (50, 52, 135d) à se fermer;
(g) un élément de poussée (78,143) ayant une extrémité proximale qui s'étend dans le châssis et une extrémité distale, l'élément de poussée étant positionné d'une manière mobile dans la section oblongue entre une position proximale et une position distale, par quoi une pince est amenée entre les mâchoires flexibles opposées;
(h) un ressort (140, 207) positionné dans le châssis et butant contre l'élément de came et l'élément de poussée, le ressort sollicitant l'élément de came proximalement et l'élément de poussée distalement; et
(i) la section endoscopique est montée sur le châssis d'une manière (132, 170) qui permet une manipulation manuelle au châssis pour faire tourner les mâchoires autour de l'axe de la section endoscopique, relativement au châssis.

2. Appareil selon la revendication 1, où ladite poignée d'actionnement est fonctionnellement reliée audit élément de came et audit élément de poussée dans ledit châssis de telle sorte qu'un mouvement de la poignée d'actionnement par rapport au châssis déplace l'élément de came et l'élément de poussée contre la sollicitation du ressort.

3. Appareil selon la revendication 1 ou 2, comprenant en outre une première bielle (120, 122) montée sur la poignée d'actionnement et l'élément de poussée, et une deuxième bielle (116, 118) montée sur la poignée d'actionnement et l'élément de came.

4. Appareil selon la revendication 3, où la première bielle est configurée pour transformer un mouvement arqué de la poignée d'actionnement par rapport au châssis en un mouvement linéaire de l'élément de poussée par rapport au châssis, et la deuxième bielle est configurée pour transformer le mouvement arqué de la poignée d'actionnement en un mouvement linéaire de l'élément de came par rapport au châssis.

5. Appareil selon l'une des revendications précédentes, où le châssis définit un manche stationnaire (100) et où la poignée d'actionnement (18) est déplaçable entre une première position espacée du manche stationnaire et une deuxième position à proximité du manche stationnaire.

6. Appareil selon la revendication 5, où ladite poignée d'actionnement est fonctionnellement reliée audit élément de came et audit élément de poussée dans ledit châssis de telle sorte qu'un déplacement de la poignée d'actionnement de la première position à la deuxième position se traduit par un déplacement de l'élément de came distalement et de l'élément de poussée proximalement par rapport au châssis.

7. Appareil selon la revendication 5 ou 6, où le ressort (140, 207) est un ressort tendeur, et le déplacement de la poignée d'actionnement de la première position à la deuxième position se traduit par un étirement du ressort tendeur.

8. Appareil selon l'une des revendications précédentes, où l'élément de poussée comporte un tube de poussoir (209) positionné d'une manière déplaçable dans le châssis et l'élément de came comporte un tube de came (212) positionné d'une manière déplaçable dans le châssis, et le ressort (207) agit sur le tube de poussoir et le tube de came, en sollicitant le tube de poussoir distalement et le tube de came proximalement.

9. Appareil selon l'une des revendications précédentes, où la section endoscopique définit un axe longitudinal et est apte à tourner par rapport au châssis, et en outre où, quelle que soit l'orientation angulaire de la section allongée relativement au châssis, le ressort conserve une orientation angulaire fixe par rapport au châssis.

10. Appareil selon l'une des revendications précédentes, comprenant en outre un bloc d'étanchéité (401) positionné dans la section oblongue et coopérant avec l'élément de came et avec l'élément de poussoir pour empêcher un écoulement des gaz à travers la section oblongue.

11. Appareil selon l'une des revendications précédentes, comprenant en outre un chemin de pinces (137) installé dans la section oblongue et une pluralité de pinces (138) positionnées dans le chemin de pinces.

12. Appareil selon l'une des revendications précédentes, comprenant en outre (i) un loquet (150) positionné dans le châssis, le loquet étant déplaçable entre une position déverrouillée et une position verrouillée où le loquet (158) vient en prise avec l'élément de poussée (134) et retient l'élément de poussée dans une position proximale contre la sollicitation du ressort, et (ii) un déclencheur (16) monté sur le châssis et déplaçable d'une première position à une deuxième position pour déplacer le loquet de la position verrouillée à la position déverrouillée.

13. Appareil selon l'une des revendications précédentes, où l'élément de poussée comporte une barre de poussée et un tube de poussoir, et où l'élément de came comporte un canal de came et un tube de came.

14. Appareil selon l'une des revendications précédentes, comprenant en outre un mécanisme pour installer détachablement la section endoscopique sur le châssis, une première partie (227) du mécanisme étant positionnée à l'extrémité proximale de la section oblongue, et une deuxième partie (226) du mécanisme étant positionnée dans le châssis.
